(19) Europäisches Patentamt
European Patent Office
Office européen des brevets

(11) Publication number: 0 121 350 B1

(12) # EUROPEAN PATENT SPECIFICATION

(45) Date of publication of patent specification: 13.05.87

(21) Application number: 84301433.3

(22) Date of filing: 05.03.84

(51) Int. Cl.[4]: C 07 C 149/20, C 07 C 149/23, C 07 K 1/06, C 07 C 149/243, A 61 K 31/10, A 61 K 31/185, A 61 K 31/16, A 61 K 37/02, C 07 D 303/38, C 07 D 303/40, C 07 D 303/46

(54) **Substituted unsaturated mercaptocarboxylic acids and derivatives as leukotriene antagonists.**

(30) Priority: 07.03.83 US 472773
07.03.83 US 472774
07.03.83 US 472772

(43) Date of publication of application:
10.10.84 Bulletin 84/41

(45) Publication of the grant of the patent:
13.05.87 Bulletin 87/20

(84) Designated Contracting States:
AT BE CH DE FR GB IT LI LU NL SE

(56) References cited:
EP-A-0 068 739

PROCEEDINGS OF THE NATIONAL ACADEMY OF SCIENCES OF THE UNITED STATES OF AMERICA, vol. 78, no. 5, May 1981, Medical Sciences Washington, US J.M. DRAZEN et al.: "Contractile activities of structural analogs of leukotrienes C and D: Necessity of a hydrophobic region", pages 3195-3198

(73) Proprietor: SMITHKLINE BECKMAN CORPORATION
P.O. Box 7929 1 Franklin Plaza
Philadelphia Pennsylvania 19101 (US)

(72) Inventor: Gleason, John Gerald
125 Dorado Drive
Delran, NJ 08075 (US)
Inventor: Hall, Ralph Floyd
9 Galston Drive
Robbinsville, NJ 08691 (US)
Inventor: Wen-Fu Ku, Thomas
1413 Southwind Way
Dresher, PA 19025 (US)

(74) Representative: Waters, David Martin, Dr. et al
Smith Kline & French Laboratories Ltd. Patent Department Mundells
Welwyn Garden City Hertfordshire AL7 1EY (GB)

EP 0 121 350 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Background of the invention

"Slow Reacting Substance of Anaphylaxis" (SRS-A) has been shown to be a highly potent bronchoconstricting substance which is released primarily from mast cells and basophils on antigenic challenge. SRS-A has been proposed as a primary mediator in human asthma. SRS-A, in addition to its pronounced effects on lung tissue, also produces permeability changes in skin and may be involved in acute cutaneous allergic reactions. Further, SRS-A has been shown to effect depression of ventricular contraction and potentiation of the cardiovascular effects of histamine.

The discovery of the naturally occurring leukotrienes and their relationship to SRS-A has reinforced interest in SRS-A and other arachidonate metabolites. SRS-A derived from mouse, rat, guinea pig and man have all been characterized as mixtures of leukotriene-$C_4$ ($LTC_4$); leukotriene-$D_4$ ($LTD_4$) and leukotriene-$E_4$ ($LTE_4$); the structural formulae of which are represented below.

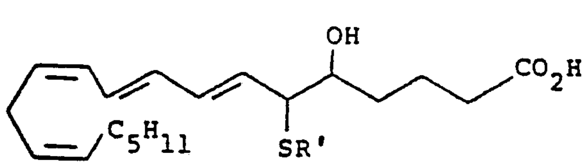

γ-Glu
$LTC_4$ R'=Cys-Gly
$LTD_4$ R'=Cys-Gly
$LTE_4$ R'=Cys

By antagonizing the effects of $LTC_4$, $LTD_4$ and $LTE_4$ or other pharmacologically active mediators at the end organ, airway smooth muscle, the compounds and pharmaceutical compositions of the instant invention are valuable in the treatment of diseases in which leukotrienes are a factor, such as asthma.

Detailed description of the invention

The compounds of this invention are represented by the following general structural formula (I)

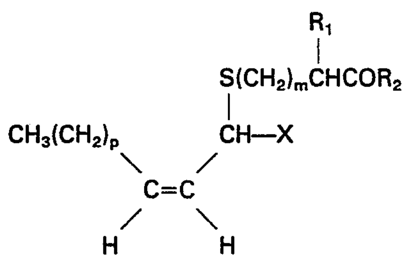

(I)

wherein m is 0, 1 or 2; p is 9, 10, 11, 12 or 13; $R_1$ is hydrogen, amino or

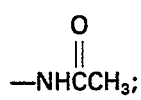

$R_2$ is hydroxyl, amino, $—NHCH_2CO_2H$,

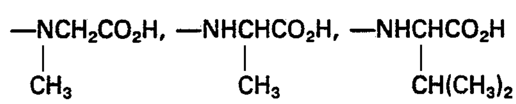

or $—NHCH_2CONH_2$; and X is $—CO_2H$, $—CH_2OH$,

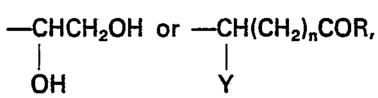

wherein n is 1 or 2, Y is hydrogen or hydroxyl and R is hydroxyl or amino with the proviso that when m is 0, $R_1$ is hydrogen, or a pharmaceutically acceptable salt thereof.

Illustrative of the compounds of formula (I) are those compounds in which the alkyl group adjacent to the double bond contains twelve carbon atoms (i.e. p is 11), which are represented by the formula (II)

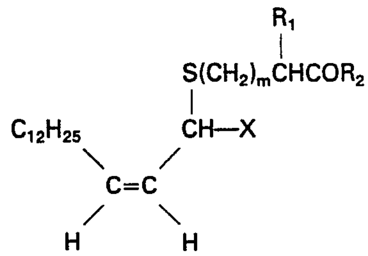

(II)

wherein m, $R_1$, $R_2$ and X are described above.

The compounds of formula (II) wherein X is $—CO_2H$ are 3(Z)-hexadecenoic acid derivatives which are exemplified by 2-[(2-carboxyethyl)thio]-3(Z)-hexadecenoic acid.

The compounds of formula (II) wherein X is $—CH_2OH$ are 3(Z)-hexadecen-1-ol derivatives which are exemplified by 2-[(2-carboxyethyl)thio]-3(Z)-hexadecen-1-ol.

The compounds of the formula (II) wherein X is

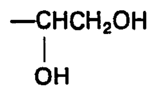

are 4(Z)-heptadecen-1,2-diol derivatives which are exemplified by 3-[(2-carboxyethyl)thio]-4(Z)-heptadecen-1,2-diol.

The compounds of formula (I) wherein X is

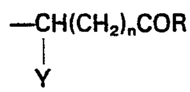

in which n, Y and R are described above are represented by the formula (III)

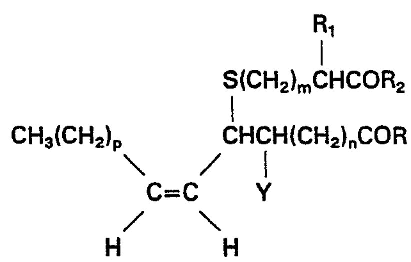

(III)

wherein m, n, p, Y, R, $R_1$, and $R_2$ are described above.

The compounds of formula (III) wherein n is 2 and p is 11 have a $C_{19}$ carbon skeleton and are designated 6(Z)-nonadecenoic acid derivatives. Similarly, when n is 1 and p is 11, the compounds of formula (III) have a $C_{18}$ carbon skeleton and are designated 5(Z)-octadecenoic acid derivatives. When n is 2 and p is 9 the compounds of formula (III) have a $C_{17}$ carbon skeleton and are designated 6(Z)-heptadecenoic acid derivatives. Also, when n is 2 and p is 13 the compounds of formula (III) have a $C_{21}$ carbon skeleton and are designated 6(Z)-heneicosenoic acid derivatives.

The 6(Z)-nonadecenoic acid derivatives of the compounds of formula (III) wherein R is hydroxyl and Y is hydroxyl contain two asymmetric centers, one of which is at carbon atom 4 (i.e. the hydroxyl substituted carbon atom) and one of which is at carbon atom 5 (i.e. the thiol substituted carbon atom). This leads to the possibility of four stereoisomers for each compound. In practice, the compounds of this invention of formula (III) have been prepared in a mixture of two stereoisomers, that is the 4R, 5S isomer and the 4S, 5R isomer. The individual pure stereoisomers are obtainable by preparative high pressure liquid chromatography (HPLC) separation of the appropriate intermediate compounds if those compounds possess a third asymmetric center. In the cases where a third asymmetric center is not available in the synthetic pathway of the desired compounds, one may be introduced by employing an asymmetric protecting group, such as an N-trifluoroethoxycarbonyl-i-prolyl ester. After separation of the individual stereoisomer, the protecting group is removed by standard procedures.

The 6(Z)-nonadecenoic acid derivatives of formula (III) are exemplified by the following compounds as the 4R, 5S isomer, the 4S, 5R isomer or mixture of the two isomers:

4-hydroxy-5-[(2-carboxyethyl)thio]-6(Z)-nonadecenoic acid;

5-[(3-carboxymethylamino-3-oxopropyl)-thio]-4-hydroxy-6(Z)-nonadecenoic acid;

5-[(2-amino-3-carboxymethylamino-3-oxopropyl)thio]-4-hydroxy-6(Z)-nonadecenoic acid;

5-[(3-carboxymethyl-N-methylamino-3-oxopropyl)-thio]-4-hydroxy-6(Z)-nonadecenoic acid;

5-[(2-amino-3-carboxamidomethylamino-3-oxo-propyl)thio]-4-hydroxy-6(Z)-nonadecenoic acid;
4-hydroxy-5-[(carboxymethyl)thio]-6(Z)-nonadecenoic acid;
5-[[2-(aminocarbonyl)ethyl]thio]-4-hydroxy-6(Z)-nonadecenoic acid;
4-hydroxy-5-[(2-amino-2-carboxyethyl)thio]-6(Z)-nonadecenoic acid; and
4-hydroxy-5-[(3-carboxypropyl)thio]-6(Z)-nonadecenoic acid.

The 5(Z)-octadecenoic acid derivatives of the compounds of formula (III) wherein Y is hydroxyl are also contain two asymmetric centers at carbon atom 3 and carbon atom 4 and thus the possibility of four stereoisomers for each compound exists.

Specific compounds of the formula (III) are those 5(Z)-octadecenoic acid derivatives exemplified by the following compounds as a mixture of the four isomers:
3-hydroxy-4-[(2-carboxyethyl)thio]-5(Z)-octadecenoic acid.

Also representative of the compounds of formula (III) is 4-[(2-carboxyethyl)thio]-5(Z)-octadecenoic acid.

The compounds of the formula (III) wherein Y is hydrogen are exemplified by 5-[(2-carboxyethyl)thio]-6(Z)-nonadecenoic acid.

Specific compounds of the formula (III) are those 6(Z)-nonadecenoic acid derivatives wherein R is amino and Y is hydroxyl are exemplified by 5-[(3-carboxymethylamino-3-oxopropyl)thio]-4-hydroxy-6(Z)-nonadecenamide and 4-hydroxy-5-[(2-carboxyethyl)thio]-6(Z)-nonadecanamide.

Additional compounds of the formula (III) are those 6(Z)-heptadecenoic acid derivatives which are exemplified as a mixture of isomers of 4-hydroxy-5-[(2-carboxyethyl)thio]-6(Z)-heptadecenoic acid.

Further compounds of the formula (III) are those 6(Z)-heneicosenoic acid derivatives wherein R is hydroxyl and X is hydroxyl which are exemplified as a mixture of isomers of 4-hydroxy-5-[(2-carboxyethyl)thio]-6(Z)-heneicosenoic acid.

The compounds of formula (I) wherein X is $-CO_2H$, $-CH_2OH$ and

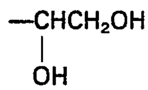

are prepared via the following synthetic pathways starting with 4-hydroxybut-2(E)-ene-1-al tetrahydropyranyl ether (1), wherein THP is a tetrahydropyranyl radical, as follows:

Pathway A

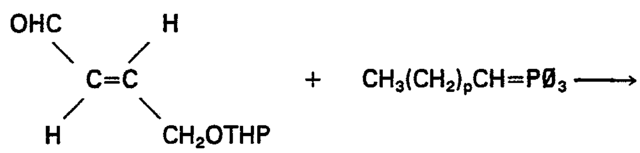


(1) (2)

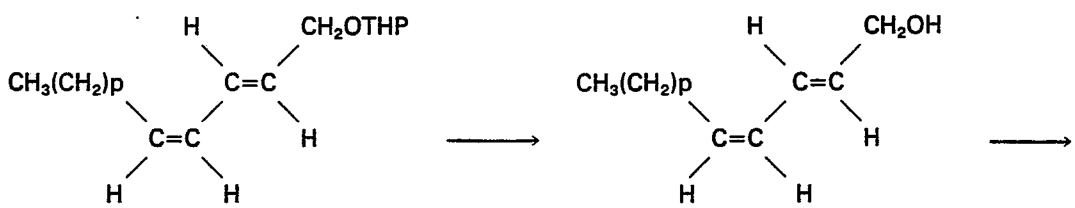

(3) (4)

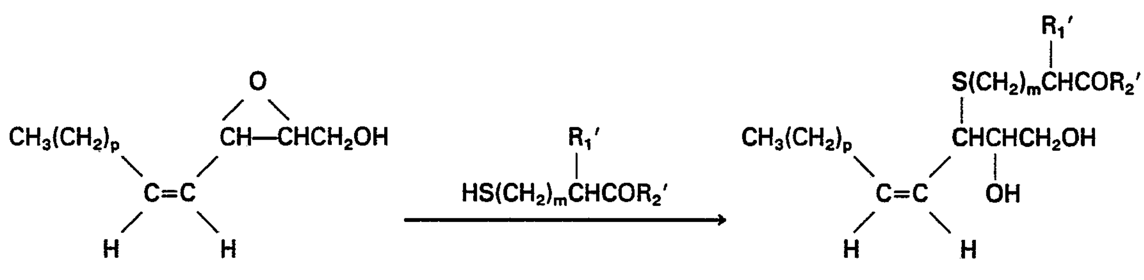

(5) (6)

Pathway B

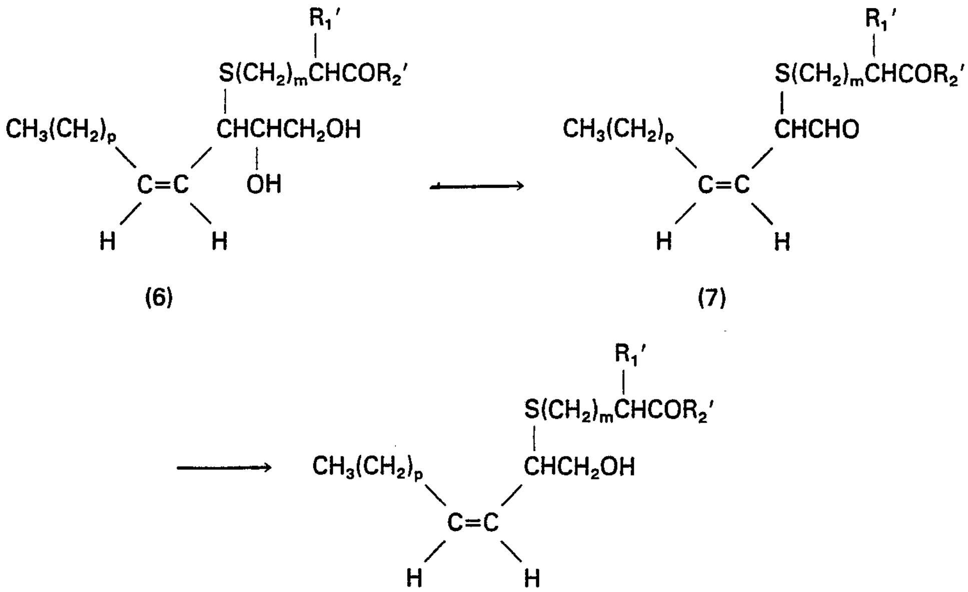

(6) (7) (8)

Pathway C

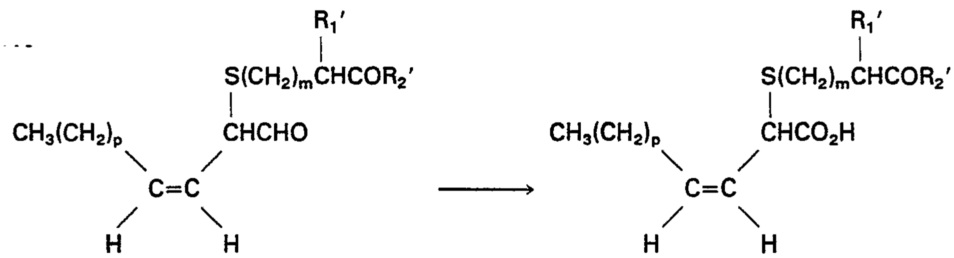

(7) (9)

Compound (1), which is a known compound, is reacted with the appropriate alkyltriphenyl phosphonium ylid under Wittig conditions to yield compound (3). Compound (3) is hydrolyzed to cleave the THP ether and gives compound (4) which is epoxidized to afford compound (5). Compound (5) is reacted with the appropriate mercaptan, wherein $R_1'$ and $R_2'$ are $R_1$ and $R_2$ respectively as described above or a radical which is easily converted into the desired substituent, such as an alkyl ester or trifluoromethylacetamide, to give compound (6). Compound (6) may either (a) be easily converted into compounds of the formula (I) wherein X is

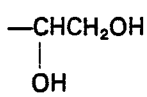

or analogs thereof; or (b) oxidatively cleaved to compound (7). Compound (7) may either (a) be reduced to compound (8) which is easily converted to compounds of the formula (I) wherein X is $-CH_2OH$ or analogs thereof or (b) oxidized to compound (9) which is easily converted into compounds of the formula (I) wherein X is $-CO_2H$ or analogs thereof.

The compounds of the formula (III) wherein n is 2 and Y is hydroxy are readily prepared by reacting the appropriate thiol containing compound of the formula (A)

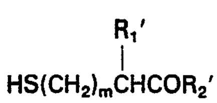 (A)

wherein m is described above and $R_1'$ and $R_2'$ are respectively $R_1$ and $R_2$ or a radical easily convertible to the desired substituent, such as an alkyl ester or trifluoromethylacetamide, with a 4,5-epoxy-6(Z)-alkenoic acid derivative of the formula (B)

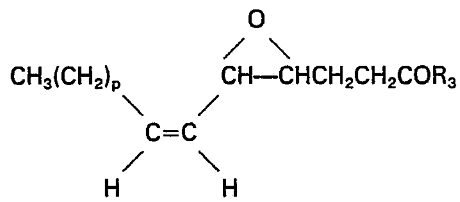

(B)

wherein p is described above and $R_3$ is R or a radical which is easily convertible into the desired acid moieties, such as $CO_2$alk wherein alk is a lower alkyl group. The thiol containing compounds of formula (A) are known or easily prepared from known compound utilizing standard chemical transformations. The 4,5-epoxy-6(Z)-alkenoic acid derivatives (B) are readily prepared from monoalkyl succinate (10) via the following synthetic pathway:

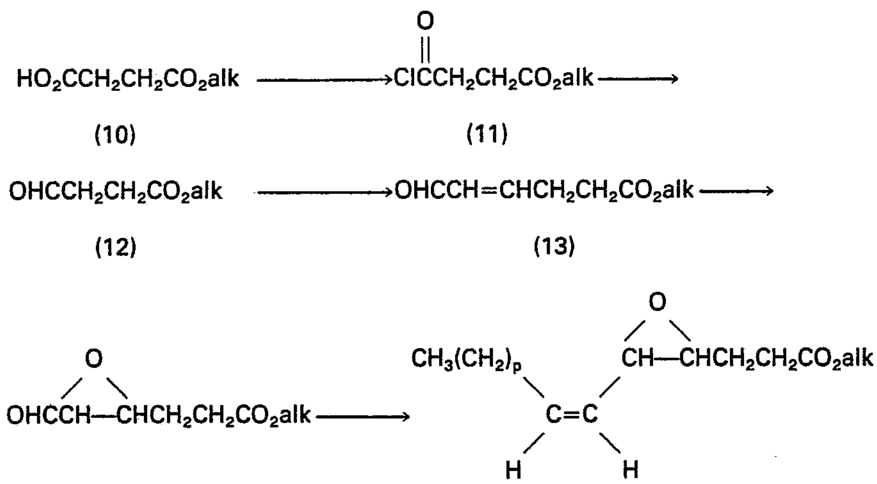

(10) (11)

(12) (13)

(14) (15)

Mono-methyl succinate (10), wherein alk is methyl, was chlorinated with oxalyl chloride in dimethyl formamide and methylene chloride to afford 3-carbomethoxypropionyl chloride (11). Compound (11) was catalytically hydrogenated over palladium-charcoal catalyst in the presence of 2,6-lutidine to yield 3-carbomethoxypropionaldehyde (12) which was reacted with formylmethylene triphenylphosphorane under Wittig reaction conditions to obtain 5-carbomethoxy-2-pentenal (13). Compound (13) was epoxidized with aqueous hydrogen peroxide in the presence of 1N sodium bicarbonate to afford methyl-4,5-epoxy-6-oxohexanoate (14). Compound (14) is reacted with alkyltriphenylphosphonium ylid to give methyl-4,5-epoxy-6(Z)-alkenoate (15).

The 5(Z)-alkenoic acid derivatives of the formula (III) wherein n is 1 and Y is hydroxy are prepared via the synthetic pathway starting from 2-[(2-carbomethoxyethyl)-thio]-3(Z)-alkenal (7) as follows:

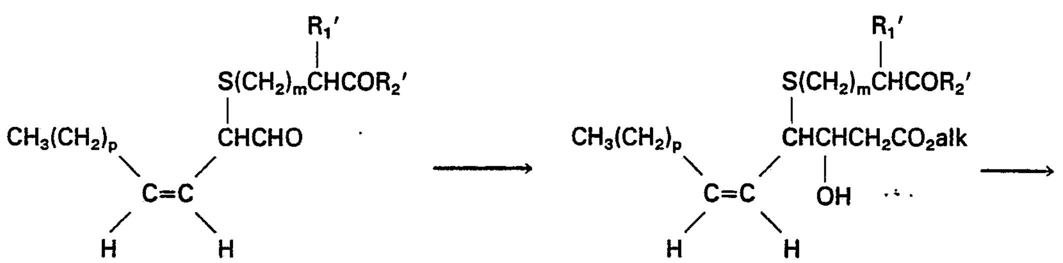

(III)

(7) (16)

Compound (7) is reacted with methyl acetate in the presence of lithium diisopropylamide to give methyl-3-hydroxy-4-[(2-carbomethoxyethyl)thiol]-5(Z)-alkenoate (16) which is saponified to afford compound of the formula (III) wherein m is 1, R is hydroxyl $R_1$ is hydrogen and $R_2$ is hydroxyl.

The 6(Z)-nonadecenoic acid derivatives of formula (III) wherein n is 2 and Y is hydrogen are prepared via the pathway starting from 5-substituted pentanoic acids (17) as follows:

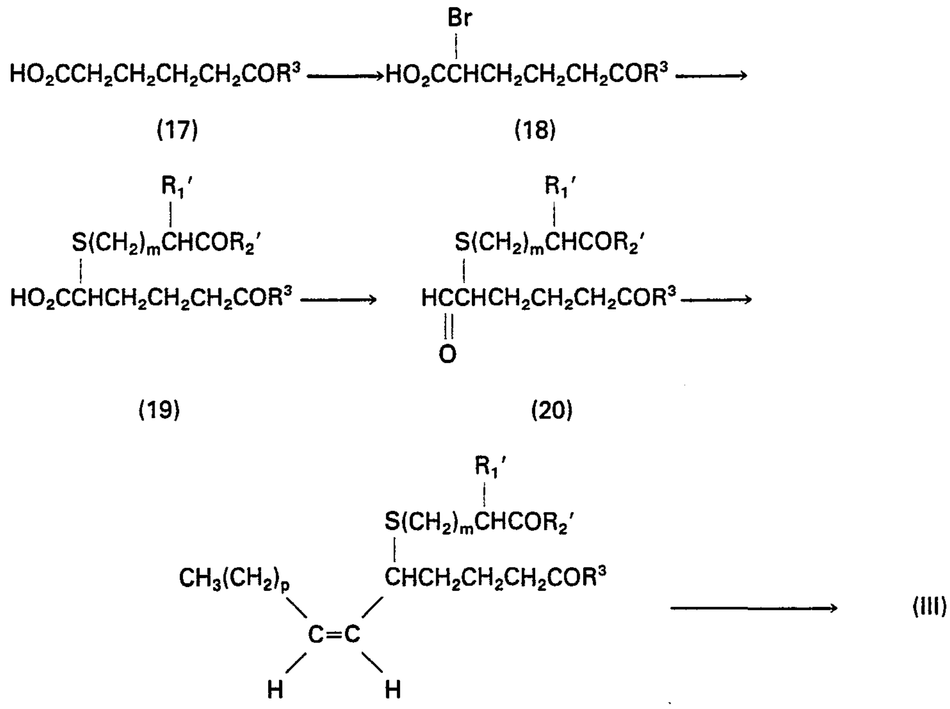

(17) (18)

(19) (20)

(21) (III)

For example, 5-carbomethoxypentanoic acid (17), wherein $R^3$ is O-methyl, was reacted with thionyl chloride and then N-bromosuccinimide (NBS) followed by base hydrolysis to give 2-bromo-5-carbomethoxypentanoic acid (18). Compound (18) was reacted with methyl-3-mercaptopropionate (m=1, $R_1'$=H and $R_2'$=$OCH_3$) to yield 5-carbomethoxy-2-[(2-carbomethoxyethyl)thio]-pentanoic acid (19). Compound (19) reacted first with diborane and then with dimethylsulfoxide and trifluoroacetic anhydride to afford 5-carbomethoxy-2-[(2-carbomethoxyethyl)thio]-pentanal (20). Compound (20) was reacted under Wittig conditions with alkyltriphenylphosphonium ylid to yield methyl-5-[(2-carbomethoxyethyl)thio]-6(Z)-alkenoate (21) which is saponified to afford a compound of the formula (III) wherein m is 1, $R_1$ is hydrogen, and $R_2$ is hydroxyl.

The 5(Z)-octadecenoic acid derivatives of the formula (III) wherein n is 1 and Y is hydrogen can be prepared via the general synthetic pathway utilized for the preparation of the 6(Z)-nonadecenoic acid derivatives but starting from 4-substituted butanoic acid.

The compounds of the formula (III) wherein R is amino can be prepared by initially forming the γ-lactone of the appropriate compound of formula (III) with trifluoroacetic anhydride and then reacting the γ-lactone with ammonia to afford the desired amide.

The instant invention also includes the 4,5-epoxy-6(Z)-alkenoic acid derivatives of the formula (B)

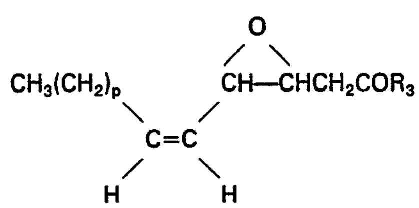

(B)

wherein p is 9, 10, 11, 12 or 13 and $R_3$ is R or a radical which is easily convertible into R such as Oalk wherein alk is an alkyl radical containing one to six carbon atoms.

Additionally, this invention includes the compounds of the formula (C)

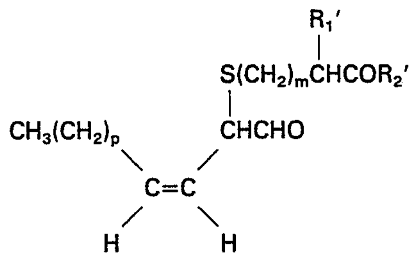

(C)

wherein m is 0, 1 or 2; p is 9, 10, 11, 12 or 13; $R_1'$ is hydrogen,

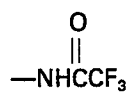 or 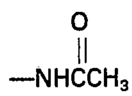

and $R_2'$ is amino, $-NHCH_2CO_2R_3'$,

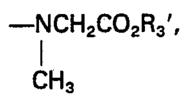, 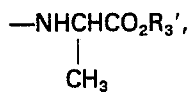, 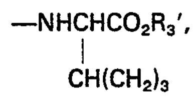,

$-NHCH_2CONH_2$ or $OR_3'$ wherein $R_3'$ is an alkyl radical containing one to six carbon atoms with the proviso that when m is 0, $R_1'$ is hydrogen.

The compounds of the formulae (B) and (C) possess the double bond in the *cis* conformation. The structural similarities between these compounds and the compounds of the formula (I) contribute to the leukotriene antagonist properties of the compounds of formula (I).

The leukotriene antagonist activity of the compounds of this invention is measured by the ability of the compound to inhibit leukotriene induced contraction of guinea pig tracheal tissues *in vitro* and to inhibit leukotriene induced bronchoconstriction in guinea pigs *in vivo*. The following methodologies were employed: *In vitro*: Guinea pig (adult male albino Hartley strain) tracheal spiral strips of approximate dimensions 2 to 3 mm cross-sectional width and 3.5 cm length were bathed in modified Krebs buffer in jacketed 10 ml tissue bath and continuously aerated with 95% $O_2$/5% $CO_2$. The tissues were connected via silk suture to force displacement transducers for recording isometric tension. The tissues were equilibrated for 1 hr., pretreated for 15 minutes with meclofenamic acid (1 μM) to remove intrinsic prostaglandin responses, and then pretreated for an additional 30 minutes with either the test compound or vehicle control. A cumulative concentration-response curve for $LTD_4$ on triplicate tissues was generated by successive increases in the bath concentration of the $LTD_4$. In order to minimize intertissue variability, the contractions elicited by $LTD_4$ were standardized as a percentage of the maximum response obtained to a reference agonist, carbachol (10 μM).

Calculations:

The averages of the triplicate $LTD_4$ concentration-response curves both in the presence and absence of the test compound were plotted on log graph paper. The concentration of $LTD_4$ needed to elicit 30% of the contraction elicited by carbachol was measured and defined as the $EC_{30}$. The $pA_2$ value for the test compound was determined by the following equations:

1. $$\frac{EC_{30}\text{ (presence of test compound)}}{EC_{30}\text{ (presence of vehicle control)}}=\text{dose ratio}=X$$

2. $K_B$=concentration of test compound/(X-1)

3. $pA_2 \cong -\log K_B$

*In vivo:* Anesthetized, spontaneously breathing guinea pigs (Adult male albino Hartley strain) were monitored on a Buxco pulmonary mechanics computer. Changes in airway resistance ($R_L$) were calculated by the computer on a breath-by-breath basis at isovolumic points from signals measuring airflow and transpulmonary pressure using differential pressure transducers. Animals received either test compound or vehicle control intravenously via the jugular vein. $LTD_4$ was then injected into the jugular vein. The bronchoconstriction produced was reflected by % changes in airways resistance relative to the baseline values obtained prior to injection of the test compound or vehicle control. Each guinea pig received either vehicle control or test compound.

Calculations:

The average of 3—6 animals per treatment was calculated using the % changes in the pulmonary

parameters for control and test compound-treated animals. The average % inhibition by the test compound was calculated from the following equation:

$$\frac{R_L\ (\text{vehicle control}) - R_L(\text{test compound})}{R_L\ (\text{vehicle control})} \times 100$$

The compounds of this invention possess biosignificant antagonist activity against leukotrienes, primarily leukotriene $D_4$. Representative of the antagonist activity of the compounds of this invention, tabulated below are a number of claimed compounds and the $pA_2$ values and the $R_L$ values calculated from the above test protocols.

Compounds of the formula (II)

| X | m | $R_1$ | $R_2$ | In vitro $pA_2$ |
|---|---|---|---|---|
| $—CO_2H$ | 1 | —H | —OH | 6.0 |
| $—CH_2OH$ | 1 | —H | —OH | 5.3 |
| $—CHCH_2OH$<br>\|<br>OH | 1 | —H | —OH | 5.2 |

Compounds of the formula (III)

| | m | n | p | Y | R | $R_1$ | $R_2$ | In vitro $pA_2$ | In vivo Concentration | $R_L$ |
|---|---|---|---|---|---|---|---|---|---|---|
| | 1 | 2 | 11 | —OH | —OH | H | —OH | 6.2 | 5 mg/kg | 91% |
| | 1 | 2 | 11 | —OH | —OH | H | $—NHCH_2CO_2H$ | 6.2 | 5 mg/kg | 36% |
| [a] | 1 | 2 | 11 | —OH | —OH | $—NH_2$ | $—NHCH_2CO_2H$ | 5.2 | 5 mg/kg | 96% |
| [b] | 1 | 2 | 11 | —OH | —OH | $—NH_2$ | $—NHCH_2CO_2H$ | 5.0[c] | — | — |
| | 1 | 2 | 11 | —OH | —OH | H | $—N(CH_3)CH_2CO_2H$ | 6.1 | 5 mg/kg | 90% |
| | 1 | 2 | 11 | —OH | —OH | $—NH_2$ | $—NHCH_2CONH_2$ | 5.5 | 5 mg/kg | 98% |
| | 0 | 2 | 11 | —OH | —OH | H | —OH | 5.9 | — | — |
| | 1 | 2 | 11 | —OH | —OH | H | $—NH_2$ | 5.1 | 5 mg/kg | 38% |
| [a,d] | 1 | 2 | 11 | —OH | —OH | $—NH_2$ | —OH | 5.1 | — | — |
| [b,e] | 1 | 2 | 11 | —OH | —OH | $—NH_2$ | —OH | 5.1 | — | — |
| | 2 | 2 | 11 | —OH | —OH | H | —OH | 6.2 | — | — |
| | 1 | 1 | 11 | —OH | —OH | H | —OH | 6.1 | 10 mg/kg | 88% |
| | 1 | 1 | 11 | H | —OH | H | —OH | 6.6±0.4 | — | — |
| | 1 | 2 | 11 | H | —OH | H | —OH | 5.5 | 10 mg/kg | 98% |
| [f] | 1 | 2 | 11 | —OH | $—NH_2$ | $—NH_2$ | $—NHCH_2CO_2H$ | 4.9 | — | — |
| [g] | 1 | 2 | 11 | —OH | $—NH_2$ | H | —OH | 5.4 | — | — |
| | 1 | 2 | 9 | —OH | —OH | H | —OH | 5.3 | — | — |
| | 1 | 2 | 13 | —OH | —OH | H | —OH | 5.4 | — | — |

[a] 4(R),5(S) isomer
[b] 4(S),5(R) isomer
[c] partial agonist
[d] 1.0 Na salt
[e] 1.5 Na salt
[f] Na salt
[g] 0.3 M $NH_3$

The specificity of the antagonist activity of a number of the compounds of this invention is demonstrated by relatively low levels of antagonism toward agonists such as potassium chloride, carbachol, histamine and $PGF_{2\alpha}$.

Pharmaceutical compositions of the present invention comprise a pharmaceutical carrier or diluent and an amount of a compound of the formula (I) or a pharmaceutically acceptable salt, such as an alkali metal salt thereof sufficient to produce the inhibition of the effects of leukotrienes, such as symptoms of asthma and other allergic diseases.

When the pharmaceutical composition is employed in the form of a solution or suspension, examples of appropriate pharmaceutical carriers or diluents include: for aqueous systems, water; for non-aqueous systems, ethanol, glycerin, propylene glycol, corn oil, cottonseed oil, peanut oil, sesame oil, liquid paraffins and mixtures thereof with water, for solid systems, lactose, kaolin and mannitol; and for aerosol systems, dichlorodifluoromethane, chlorotrifluoroethane and compressed carbon dioxide. Also, in addition to the pharmaceutical carrier or diluent, the instant compositions may include other ingredients such as stabilizers, antioxidants, preservatives, lubricants, suspending agents, viscosity modifiers and the like, provided that the additional ingredients do not have a detrimental effect on the therapeutic action of the instant compositions.

The nature of the composition and the pharmaceutical carrier or diluent will, of course, depend upon the intended route of administration, i.e. parenterally or by inhalation.

In general, particularly for the prophylactic treatment of asthma, the compositions will be in a form suitable for administration by inhalation. Thus the compositions will comprise a suspension or solution of the active ingredient in water for administration by means of a conventional nebulizer. Alternatively, the compositions will comprise a suspension or solution of the active ingredient in a conventional liquified propellant or compressed gas to be administered from a pressurized aerosol container. The compositions may also comprise the solid active ingredient diluted with a solid diluent for administration from a powder inhalation device. In the above compositions, the amount of carrier or diluent will vary but preferably will be the major proportion of a suspension or solution of the active ingredient. When the diluent is a solid it may be present in less, equal or greater amounts than the solid active ingredient.

For parenteral administration the pharmaceutical composition will be in the form of a sterile injectable liquid such as an ampul or an aqueous or nonaqueous liquid suspension.

Usually a compound of formula I is administered to an animal subject in a composition comprising a nontoxic amount sufficient to produce an inhibition of the symptoms of an allergic response. When employed in this manner, the dosage of the composition is selected from the range of from 350 mg to 700 mg of active ingredient for each administration. For convenience, equal doses will be administered 1 to 4 times daily with the daily dosage regimen being selected from about 350 mg to about 2800 mg.

The pharmaceutical preparations thus described are made following the conventional techniques of the pharmaceutical chemist as appropriate to the desired end product.

Included within the scope of this disclosure is the method of inhibiting the symptoms of an allergic response resulting from a mediator release which comprises administering to an animal subject a therapeutically effective amount for producing said inhibition of a compound of formula I, preferably in the form of a pharmaceutical composition. The administration may be carried out in dosage units at suitable intervals or in single doses as needed. Usually, this method will be practiced when relief of allergic symptoms is specifically required, however, the method is also usefully carried out as continuous or prophylactic treatment. It is within the skill of the art to determine by routine experimentation the effective dosage to be administered from the dose range set forth above, taking into consideration such factors as the degree of severity of the allergic condition being treated, and so forth.

The following examples illustrate the preparation of the compounds of this invention and their incorporation into pharmaceutical compositions and as such are not to be considered as limiting the invention set forth in the claims appended hereto.

Example 1

Preparation of 3-[(2-carboxyethyl)thio]-4(Z)-heptadecen-1,2-diol

(a) Heptadec-2(E),4(Z)-dienyl tetrahydropyranyl ether 1(a)(1)
Heptadec-2(E),4(E)-dienyl tetrahydropyranyl ether 1(a)(2)

Tridecyltriphenyl phosphonium bromide (189 g, 0.3 mole) was dissolved in 900 ml of tetrahydrofuran and cooled to 0° in an ice-salt bath while stirring under argon. A 2.2 *N* solution of n-butyllithium in hexane (250 ml, 0.36 mole) was added dropwise over a period of 30 minutes. The mixture was stirred for an additional 20 minutes and then cooled to −70° in a dry ice-acetone bath. The 4-hydroxybut-2(E)-ene-1-al tetrahydropyranyl ether (51 g, 0.3 mole in 225 ml of tetrahydrofuran was added dropwise over a period of 35 minutes and the mixture stirred for an additional hour at −70°. The mixture was then poured into 6.25 liters of ether and stirred for 20 minutes. The resulting mixture was filtered through glass fiber filter paper. The filtrate was evaporated and the residue triturated with hexane, filtered and evaporated to give a 3:1 mixture of 1(a)(1); 1(a)(2).

(b) Heptadec-2(E),4(Z)-dien-1-ol 1(b)(1)
Heptadec-2(E),4(E)-dien-1-ol 1(b)(2)

The mixture of compounds 1(a)(1) and 1(a)(2) (80 g, 0.24 mole) was dissolved in 3 liters of methanol

and the pyridine p-toluenesulfonic acid (3 g, 0.012 mole) was added to the mixture stirring under argon at room temperature. The progress at the reaction was monitored by tlc. When the reaction was complete the solvent was evaporated and the residue flash chromatographed on 500 grams of silica gel eluted with 10% ethyl acetate in hexane to give a 3:1 mixture of 1(b)(1):1(b)(2). Separation of 1(b)(1) from 1(b)(2) was accomplished by careful chromatography on silica gel. Compound 1(b)(1) mp 34°—37°. Compound 1(b)(2) mp 51—55°.

(c) *Trans*-2,3-epoxy-heptadec-4-Z-ene-1-ol 1(c)

Compound 1(b)(1) (2.52 g, 10 mmol) was dissolved in 100 ml of methylene chloride stirring at room temperature under argon. A 0.5 N solution of sodium bicarbonate (30 ml) was added. The 85% m-chloroperbenzoic acid (2.03 g, 10 mmol) was added slowly in small portions. The mixture was stirred for 1.5 hours after the addition was complete. The phases were separated and the aqueous phase washed with methylene chloride. The combined organic phases were dried over anhydrous sodium sulfate filtered and evaporated. The residue was flash chromatographed on 100 grams of silica gel eluted with 10—20% ethyl acetate-hexane to give compound 1(c).

(d) 3-[(2-Carbomethoxyethyl)thio]-4(Z)-heptadecen-1,2-diol 1(d)

Compound 1(c) (7.2 g, 26.9 mmol) was dissolved in 40.2 ml of methanol containing 2% triethylamine. This solution was stirred at room temperature under argon and a solution of methyl-3-mercaptopropionate (4.92 ml, 44.4 mmol) and triethylamine (11.16 ml, 80.2 mmol) in 40.2 ml of methanol was added dropwise over a period of 15 minutes. The mixture was stirred for 5 hours at room temperature and then placed in the refrigerator overnight. The solvents were evaporated and the residue flash chromatographed on 500 grams of silica gel eluted with 10—50%, ethyl acetate in hexane to give compound 1(d), mp. 33—36°.

(e) 3-[(2-Carboxyethyl)thio]-4(Z)-heptadecen-1,2-diol 1(e)

Compound 1(d) (500 mg, 1.29 mmol) was dissolved in 2 ml of methanol and stirred under argon. A 1*N* solution of sodium hydroxide (0.65 ml, 0.65 mmol) was added dropwise and the mixture stirred for 0.5 hours at room temperature. An addition of 0.65 ml of a 1*N* NaOH solution was added and the mixture stirred for 1 hour. TLC indicated that some starting material still remained so an additional 0.2 ml of a 1*N* NaOH solution was added and the mixture stirred for an additional 1.5 hours. The methanol was stripped off and the residue acidified with dilute hydrochloric acid. The mixture was extracted with diethyl ether and the organic phase dried with anhydrous magnesium sulfate, filtered, and evaporated to give crude product. This was recrystallized from diethyl ether-hexane to give the desired compound, mp 76—77°.

| | Theory | Found |
|---|---|---|
| C | 64.13 | 64.39 |
| H | 10.23 | 10.22 |
| S | 8.56 | 8.78 |

Example 2

Preparation of 2-[(2-carboxyethyl)thio]-3(Z)-hexadecen-1-ol

(a) 2-[(2-Carbomethoxyethyl)thio]-3(Z)-hexadecen-1-al 2(a)

Compound 1(d) (2 g, 5.15 mmol) was dissolved in 10 ml of diethyl ether and stirred in a room temperature water bath. A saturated solution (100 ml) of periodic acid in diethyl ether was added in a single portion. The resulting mixture was stirred for two minutes and then immediately flash chromatographed on 150 g of silica gel with 10% ethyl acetate in hexane to give compound 2(a).

(b) 2-[(2-Carbomethoxyethyl)thio]-3(Z)-hexadecen-1-ol

Compound from Example 2(a) (192 mg, 0.5 mmol) was dissolved in 1 ml of methanol and stirred at 0° under argon. Sodium borohydride (38 mg, 1 mmol) was added and the mixture stirred at 0° for 5 minutes. The mixture was acidified with dilute hydrochloric acid and evaporated. The residue was taken up in diethyl ether and washed with dilute hydrochloric acid, aqueous sodium bicarbonate, dried over anhydrous sodium sulfate, filtered and evaporated to give crude product which was flash chromatographed on 20 grams of silica gel eluted with 15% ethyl acetate in hexane to give the above-noted compound.

(c) 2-[(2-Carboxyethyl)thio]-3(Z)-hexadecen-1-ol

Compound from Example 2(b) (180 mg, 0.5 mmol) was dissolved in 1 ml of methanol and a 1*N* solution of sodium hydroxide (1 ml, 1 mmol) was added. The mixture was stirred at room temperature under argon for 3 hours and then placed in the freezer over the weekend after which time it was stirred for an additional 2 hours at room temperature until tlc indicated only a trace of starting material remained. The methanol was stripped off and the residue acidified with dilute hydrochloric acid and extracted with chloroform. The organic phase dried over anhydrous magnesium sulfate filtered and evaporated to give crude product. This was recrystallized from hexane at −78°C to give the desired compound, mp. 39°—40°.

| | Theory | Found |
|---|---|---|
| C | 66.23 | 66.47 |
| H | 10.53 | 10.77 |
| S | 9.30 | 9.15 |

Example 3
Preparation of 2-[(2-carboxyethyl)thio]-3(Z)-hexadecenoic acid
(a) 2-[(2-Carbomethoxyethyl)thio]-3(Z)-hexadecenoic acid 3(a)(1)
Methyl-2-[(2-carbomethoxyethyl)thio]-3(Z)-hexadecenoate 3(a)(2)

Compound from Example 2(a), (0.8 g, 2.25 mmol) was dissolved in 5.5 ml of acetone. The solution was cooled to −40°C and stirred under argon. Jones reagent (0.3 ml, 0.6 mmol) was added and the mixture stirred between −30° and −40°C for 30 minutes. Additional Jones reagent (0.3 ml, 0.6 mmol) was added and the mixture stirred for 1 hour. The remaining Jones reagent (0.145 ml, 0.29 mmol) was added. Thirty minutes later the reaction which had been maintained in a temperature range of −30° to −40°C throughout the reaction was quenched by the addition of 1 ml of isopropanol. The solvents were stripped off and the residue partitioned between diethyl ether and $H_2O$. The aqueous phase was extracted with diethyl ether. The combined organic phases were dried over anhydrous $MgSO_4$ filtered and evaporated to give the crude product 3(a)(1). In order to facilitate purification, compound 3(a)(1) was treated with excess diazomethane in diethyl ether at 0° and allowed to warm to room temperature. The solvents were evaporated and the residue flash chromatographed on 200 grams of silica gel eluted with 5% EtOAc-hexane to give the desired product 3(a)(2).

(b) 2-[(2-Carboxyethyl)thio]-3(Z)-hexadecenoic acid

Compound 3(a)(2) (0.16 g, 0.41 mmol) was dissolved in 3.2 ml of methanol and stirred under argon at 0°C. A 1*N* solution of sodium hydroxide (1.6 ml, 1.6 mmol) was added and the ice bath removed. The mixture was stirred at room temperature for 2·1/2 hours. The methanol was evaporated and the residue cooled in an ice bath and acidified with dilute HCl. The aqueous phase was extracted twice with diethyl ether. The combined ether extracts were dried over anhydrous sodium sulfate, filtered and evaporated to give crude product. This was recrystallized from diethyl ether-hexane to give the desired compound, mp 52—54°C. Anal. Calcd. C: 63.65; H; 9.56; S: 8.94; Found C: 63.59; H: 9.45; S: 9.24.

Example 4
Preparation of 5-[(2-amino-3-carboxymethylamino-3-oxopropyl)thio]-4-hydroxy-6(Z)-nonadecenoic acid as the 4(R),5(S) isomer, 4(S),5(R) isomer and the mixture of the two isomers.
(a) 3-Carbomethoxypropionyl chloride 4(a)

To an ice-cold solution of mono-methyl succinate, (150 g, 1.135 mol) in 900 ml of methylene chloride and 3 ml of N,N-dimethylformamide was added 119 ml (1.26 mol) of oxalyl chloride, while keeping the pot temperature below 5°C. After the addition was complete, the reaction mixture was stirred at 0°C for 1 hour and then concentrated in vacuo to give a crude yellow liquid, which was used without purification in the Rosenmund reduction.

(b) 3-Carbomethoxypropionaldehyde 4(b)

To the acid chloride, 4(a) (8.9 g, 0.059 mol) in 200 ml of sieve-dried tetrahydrofuran was added 6.9 ml (0.059 mol) of 2,6-lutidine. After standing at room temperature for 30 minutes, the mixture was filtered and 0.7 g of 10% palladium on carbon was added to the filtrate. The mixture was hydrogenated at 50 psi for 2 hours, the solids were filtered off and the filtrate concentrated. The residue was dissolved in methylene chloride, washed twice with 10% hydrochloric acid solution and then twice with 5% sodium bicarbonate solution. The organic extract was dried with anhydrous sodium sulfate and concentrated in vacuo. Kugelrohr distillation (40—55°C/.05 mm) yielded a colorless liquid.

(c) 5-Carbomethoxy-2-pentenal 4(c)

To a mechanically-stirred solution of 4(b) (72.1 g, 0.620 mol) in 750 ml of toluene under argon was added 235.9 g (0.776 mol) of formylmethylene triphenylphosphorane. The reaction mixture was refluxed for 1.5 hours, cooled to room temperature and then concentrated in vacuo. The residue was left standing under diethyl ether at 0°C overnight. The mixture was filtered and the solid was washed with cold ether. The filtrate was concentrated and the resulting maroon oil was subjected to Kugelrohr distillation. The product was collected at 65—80°C/.05 mm.

(d) Methyl 4,5-epoxy-6-oxohexanoate 4(d)

To a solution of 36.4 ml of a 30% hydrogen peroxide solution and 58.2 ml of 1N sodium bicarbonate in 800 ml of methanol and 400 ml of water under argon was added dropwise over 45 minutes 41.2 g (0.29 mol) of 4(c) in 400 ml of methanol. After the addition was complete, the reaction was stirred for 2.5 hours at room temperature, while maintaining the pH between 9 and 9.5 by the addition of sodium bicarbonate

solution. The reaction mixture was then poured into one liter of saturated ammonium sulfate solution, the methanol was removed in vacuo, the solids were filtered off and the product was extracted into methylene chloride. The aqueous layer was back extracted twice, the combined extracts were dried with anhydrous sodium sulfate and then concentrated to give a crude golden oil. The product was Kugelrohr distilled at 82—9°C/0.1 mm.

(e) Methyl 4,5-epoxy-6(Z)-nonadecenoate 4(e)

To an ice-cold solution of tridecyl triphenylphosphonium bromide (preparation described below) (97.2 g, 0.185 mol) in 600 ml of sieve-dried tetrahydrofuran under argon was added dropwise 17.1 ml of a 2.4 M solution of n-butyl lithium in hexane; the temperature was maintained at 0°C during the 30 minute addition. The reaction mixture was stirred at this temperature for an additional 15 minutes, cooled to −78°C (dry ice/isopropanol) and then 26.5 g (0.168 mol) of 4(d) in 150 ml of dried tetrahydrofuran was added dropwise over 30 minutes. After the addition was complete, the reaction was stirred for 1 hour at −78°C and then concentrated in vacuo at 24°C. The residue was triturated with hexane and then left standing at 0°C overnight. The hexane was decanted and then the residue was sonicated four times with hexane. The combined extracts were concentrated in vacuo and the crude product purified by preparative HPLC (2 Waters Prepak silica columns, eluting with 8% ethyl acetate in hexane), giving 4(e) as an oil.

(f) Tridecyl triphenyl phosphonium bromide

A solution of 1-bromotridecane (100 g, 0.4 mol) and triphenyl phosphine (100 g, 0.4 mol) in 500 ml of xylenes was refluxed overnight. The reaction mixture was then cooled to room temperature and poured into diethyl ether. The resulting oil was washed three times with ether, dissolved in methylene chloride and then concentrated in vacuo. The oil was left standing in diethyl ether at 0°C overnight and then concentrated in vacuo to give a white solid.

(g) 4-Hydroxy-5-[(2-trifluoroacetamido-3-carbomethoxymethylamino-3-oxopropyl)thio]-6(Z)-nonadecenoic acid, γ-lactone and methyl ester

To the epoxide, 4(e) (12.9 g, 0.04 mol) under argon at room temperature was added dropwise over 1 hour a solution of 14.5 g (0.05 mol) of 2-trifluoroacetamido-3-carbomethoxymethylamino-3-oxopropylmercaptan

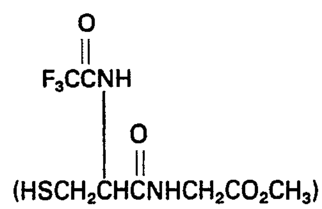

in triethylamine (6.7 ml)/methanol (150 ml). The reaction mixture was stirred at room temperature overnight and then concentrated in vacuo. The residue was dissolved in a minimum volume of methylene chloride, 100 ml, of hexane was added and then this solution was stored at −15°C for 1 hour. The resulting solid was filtered off and then the filtrated was concentrated in vacuo, to give a crude oil, consisting of a mixture of the desired products.

(h) 4(R) - Hydroxy - 5(S) - [(2 - trifluoroacetamido - 3 - carbomethoxymethylamino - 3 - oxopropyl)-thio]-6(Z)-nonadecenoic acid γ-lactone and 4(S)-Hydroxy-5(R)-[(2-trifluoroacetamido-3-carbomethoxymethylamino-3-oxopropyl)-thio]-6(Z)-nonadecenoic acid, γ-lactone

The crude mixture from Example 4(g) (20.4 g) in 200 ml of toluene was heated to 80°C in the presence of 75 mg of p-toluenesulfonic acid for 15 minutes. The reaction mixture was then concentrated in vacuo and the resulting crude oil was purified by preparative HPLC (2 Waters Prepak silica columns, eluting with 45% ethyl acetate in hexane) to give the desired products as the pure 4(R),5(S) isomer and the pure 4(S), 5(R) isomer.

(i) 4(R)-Hydroxy-5(S)-[(2-amino-3-carboxymethylamino-3-oxopropyl)thio]-6(Z)-nonadecenoic acid, hydrate

The lactone of Example 4(h) in the 4(R), 5(S) isomer form, (1.9 g, 0.00327 mol) in aqueous sodium hydroxide (0.72 g, 0.018 mol in 50 ml water) was stirred at room temperature overnight. The pH of the reaction mixture was then adjusted to 3.5 with concentrated hydrochloric acid and the resulting solid was collected and dried: mp 144—146°C; Anal. Calcd. for $C_{24}H_{44}N_2O_6S \cdot 1\ 1/4\ H_2O$; C, 56.38; H, 9.17; N, 5.48. Found: C, 56.16; H, 8.83; N, 5.54.

Similarly, the lactone of Example 1(h) in the 4(S), 5(R) isomer form was converted to the desired 4(S), 5(R) isomer of the above noted compound: mp 141—143°C; Anal. Calcd. for $C_{24}H_{44}N_2O_6S \cdot 1\ 1/4\ H_2O$: C, 56.39; H, 9.17; N, 5.48; Found: C, 56.20; H, 8.88; N, 5.18.

The isomeric mixture of the desired product was obtained treating the mixture of products prepared

according to Example 1(g) without separation as described above: mp 140—143°C; Anal. Calcd for $C_{24}H_{44}N_2O_6S \cdot 1\ 1/4\ H_2O$: C, 56.39; H, 9.17; N, 5.48; Found: C, 56.44; H, 8.75; N, 5.16.

Example 5

Preparation of 4-hydroxy-5-[(2-carboxyethyl)thio]-6(Z)-nonadecenoic acid as a mixture of the 4(R),5(S) and the 4(S),5(R) isomers.

A solution of 1.32 g (4.1 mmol) of 4,5-epoxy-6(Z)-nonadecenoic acid methyl ester and 1.46 g (12.2 mmol) of methyl-3-mercaptopropionate in 20 ml of methanol was treated with 1.62 g (16.1 mmol) of triethylamine in 10 ml of methanol under an argon atmosphere overnight. The reaction mixture was concentrated in vacuo and purification was carried out by chromatography on silica gel with hexane/ether (60/40) to give diester which contained some mono ester/lactone as evidenced by IR and tlc ($CH_2Cl_2$/hexane/acetone, 47/47/5).

A solution of this diester/lactone mixture (1.6 g, 3.6 mmol) in 30 ml of methanol was treated overnight with 1.43 g (36 mmol) of sodium hydroxide in 5 ml of water at room temperature, under an argon atmosphere. The reaction mixture was partly concentrated in vacuo, redissolved in 20 ml of water, and acidified with dilute phosphoric acid. The aqueous solution was extracted 3 times with 50 ml of ether, and the extracts were dried over magnesium sulfate, filtered and concentrated in vacuo to give white crystalline solid; m.p. 78—80°C, Anal. calcd. for $C_{22}H_{40}O_5S$: C, 63.43, H, 9.68. Found: C, 63.11, H, 9.69.

Example 6

Preparation of 5-[(3-carboxymethylamino-3-oxo-propyl)thio]-4-hydroxy-6(Z)-nonadecenoic acid

The above named compound was prepared by the general method of Example 5 using 3-carboxymethylamino-3-oxopropyl mercaptan, which in turn was prepared by the coupling reaction between 3,3′-dithio-dipropionic acid and glycine methyl ester using DCC, followed by the reduction of the disulfide with tri-n-octyl phosphine in acetone/water 1:1 mixture. Product was obtained as an oil. Anal. calcd. for $C_{24}H_{43}NO_6S$: C, 60.85; H, 9.15, N, 2.95. Found: C, 60,65; H, 9.07; N, 2.89.

Example 7

Preparation of 5-[(3-carboxymethyl-N-methylamino-3-oxopropyl)thio]-4-hydroxy-6(Z)-nonadecenoic acid

The above named compound was prepared by the general method of Example 5 using 3-carboxymethyl-N-methylamino-3-oxopropyl mercaptan. Mercaptan was prepared by coupling reaction between p-methoxy benzylthiopropionic acid and N-methyl glycine methyl ester in the presence of DCC, followed by treatment with HF at −78°C. Product obtained had a melting point of 80—82°C. Anal. calcd.: C, 61.57; H, 9.30; N, 2.87. Found: C, 61.55, H, 9.65; N, 3.02.

Example 8

Preparation of 5-[(carboxymethyl)thio]-4-hydroxy-6(Z)-nonadecenoic acid

The above named compound was prepared by the general method of Example 5 using commercial methyl thioglycolate: mp 50—52°C. Anal. calcd. for $C_{21}H_{38}O_5S$: C, 62.65; H, 9.51. Found: C, 62.74; H, 9.57.

Example 9

Preparation of 5-[(3-carboxypropyl)thio]-4-hydroxy-6(Z)-nonadecenoic acid

The above named compound was prepared by the general method of Example 5 using commercial 4-mercaptobutyric acid, mp 56—58°C. Anal. calcd. for $C_{23}H_{42}O_5S \cdot 1/4\ H_2O$: C, 63.48; H, 9.96. Found: C, 63.29; H, 9.75.

Example 10

Preparation of 5-[[2-(aminocarbonyl)ethyl]thio]-4-hydroxy-6(Z)-nonadecenoic acid

The above named compound was prepared by the general method of Example 5 using 2-(aminocarbonyl)ethyl mercaptan. Mercaptan was prepared by treatment of 3,3′-dithiodipropionyl iodide with concentrated ammonium hydroxide followed by the reduction of disulfide with tri-n-octylphosphine and acetone/water 1:1 mixture. Product obtained had a melting point of 84—86°C. Anal. calcd. for $C_{22}H_{41}NO_4S$: C, 63.57; H, 9.94. Found: C, 63,56; H, 9.82.

Example 11

Preparation of 4-hydroxy-5-[(2-amino-2-carboxyethyl)thio]-6(Z)-nonadecenoic acid as the 4(R),5(S) isomer and the 4(R),5(S) isomer

(a) 4-Hydroxy-5-[(2-trifluoroacetamido-2-carbomethoxyethyl)thio]-6(Z)-nonadecenoic acid, methyl ester and γ-lactone

To the epoxide, methyl-4,5-epoxy-6(Z)-nonadecenoate (4e) (0.9 g, 0.00277 mol) under argon at room temperature was added dropwise a solution of 1.2 g (0.00519 mol) of 2-trifluoroacetamido-2-carbomethoxyethylmercaptan in triethylamine (0.88 ml)/methanol (15 ml). The reaction mixture was stirred at room temperature for 30 hours, concentrated in vacuo and then filtered

through a silica gel bed, eluting the product mixture with chloroform. The chloroform wash was concentrated to give a mixture of the above-noted methyl ester and γ-lactone.

(b) 4-Hydroxy-5-[(2-trifluoroacetamido-2-carbomethoxyethyl)thio]-6(Z)-nonadecenoic acid, γ-lactone

The crude mixture (2 g) of the methyl ester and γ-lactone from Example 11(a) in 75 ml of toluene was heated to 80°C in the presence of 27 mg of p-toluenesulfonic acid for 20 minutes. The reaction mixture was then concentrated in vacuo and the residue was taken up in methylene chloride, washed with 5% sodium bicarbonate solution, dried with brine and anhydrous sodium sulfate and then concentrated in vacuo. The resulting crude oil was applied to the Water's Preparative HPLC (2 Prepak silica columns, eluting with 25% ethyl acetate in hexane) to give the desired products as the 4(R), 5(S) isomer and the 4(S),5(R) isomer. Each individual diastereomer was purified further on a silica "flash" column, eluting with 30% ethyl acetate in hexane.

(c) 4(R)-Hydroxy-5(S)-[(2-amino-2-carboxyethyl)thio]-6(Z)-nonadecenoic acid, sodium salt

A partial suspension of 4(R),5(S) isomer of the γ-lactone of Example 11(b) (0.3 g, 0.00057 mol) in aqueous sodium hydroxide (0.205 g, 0.004 mol in 13 ml water) was stirred for 24 hours at room temperature. The pH of the reaction mixture was then adjusted to 3.5 with concentrated hydrochloric acid to give the desired product: mp 168—170°C. Anal. calcd. for $C_{22}H_{41}NO_5S$ 1Na (−1H): C, 58.12; H, 8.87; N, 3.08. Found: C, 57.99; H, 8.85; N, 3.70.

(d) 4(S)-Hydroxy-5(R)-[(2-amino-2-carboxyethyl)thio]-6(Z)-nonadecenoic acid, sodium salt

The reaction was carried out as described in Example 11(c), to give the desired product: mp 159—161°C. Anal. Calcd. for $C_{22}H_{41}NO_5S \cdot 1.5$ Na (−1.5 H): C, 56.69; H, 8.54; N, 3.01. Found: C, 56.40; H, 8.19; N, 3.33.

Example 12

Preparation of 5[ - (2 - amino - 3 - carboxamidomethylamino - 3 - oxopropyl)thio] - 4 - hydroxy - 6(Z) - nonadecenoic acid

(a) 4(R)-Hydroxy-5(S)-[(2-trifluoroacetamido-3-carbamoylmethylamino-3-oxopropyl)thio]-6(Z)-nonadecenoic acid, γ-lactone-4(R)-hydroxy-5(S)-[(2-trifluoroacetamido-3-carbamoylmethylamino-3-oxopropyl)thio]-6(Z)-nonadecenamide

A mixture of lactone methyl ester prepared according to Example 4(g) (0.41 g, 0.7 mmol) in 10 ml of dimethoxyethane, 10 ml of methanol, and 20 ml of concentrated ammonium hydroxide solution was stirred at 0°C for 2 hours. The mixture was neutralized in the cold with concentrated hydrochloric acid to pH 7.5. The crude product was partitioned into methylene chloride. The aqueous phase was further extracted 2×40 ml methylene chloride. The combined extracts were washed with saturated sodium chloride solution, dried over sodium sulfate, and evaporated to give oil residue. Flash column chromatography (silica gel, 1.5"×6", 3% $CH_3OH/CHCl_3$, 25 ml fraction) gave the desired products. The 6(Z)-nonadecenamide had the following properties: mp 143—5°C; Anal. calcd. for $C_{26}H_{45}F_3N_4O_5S$: C, 53.59; H, 7.78; N, 9.61. Found: C, 53,86; H, 7.52; N, 9.83.

(b) 4(R)-Hydroxy-5(S)-[(2-amino-3-carbamoylmethylamino-3-oxopropylthio]-6(Z)-nonadecenoic acid

A mixture of 120 mg (0.2 mmol) of the γ-lactone of Example 12(a) in 5 ml of 0.2M sodium hydroxide solution was stirred at room temperature for 18 hours. The mixture was acidified to pH 3 by adding a concentrated hydrochloric acid solution in ice-bath. The resulting precipitates were filtered and washed quickly with cold water and dried at 56°C for 24 hours to give the desired product, mp. 143—5°C; Anal. calcd.: C, 57.51; H, 9.35; N, 8.38; Found C, 57.50; H, 9.42; N, 6.72.

Example 13

Preparation of 3-hydroxy-4-[(2-carboxyethyl)thio]-5(Z)-octadecenoic acid

(a) Methyl 3-(1,2-dihydroxyheptadec-4(Z)-enyl)thiopropionate 13(a)

Compound 1(c) (7.2 g, 26.9 mmol) was dissolved in 40.2 ml of methanol containing 2% triethylamine. This solution was stirred at room temperature under argon and a solution of methyl 3-mercaptopropionate (4.92 ml, 44.4 mmol) and triethylamine (11.16 ml, 80.2 mmol) in 40.2 ml of methanol was added dropwise over a period of 15 minutes. The mixture was stirred for 5 hours at room temperature and then placed in the refrigerator overnight. The solvents were evaporated and the residue flash chromatographed on 500 grams of silica gel eluted with 10—50% ethyl acetate in hexane to give compound 13(a), mp 33—36°.

(b) 2-[(2-Carbomethoxyethyl)thio]hexadec-3(Z)-enal 13(b)

Compound 13(a) (2 g, 5.15 mmol) was dissolved in 10 ml of diethyl ether and stirred in a room temperature water bath. A saturated solution (100 ml) of periodic acid in diethyl ether was added in a single portion. The resulting mixture was stirred for two minutes and then immediately flash chromatographed on 150 g of silica gel with 10% ethyl acetate in hexane to give compound 13(b).

(c) Methyl 3-hydroxy-4-[(2-carbomethoxyethyl)thio]octadec-5(Z)-enoate 13(c)

A dry flask sealed with a septum and maintained under an argon atmosphere was charged with 4.5 ml of hexane and cooled in an ice bath. A 2.2 *M* solution of n-BuLi (1.03 ml, 2.25 mmol) was added followed by the dropwise addition of diisopropyl amine (0.315 ml, 2.25 mmol). The solution was stirred at 0°C for 10 minutes and then cooled to −78°C in a dry ice-acetone bath for 15 minutes. A solution of methyl acetate (0.18 ml, 2.25 mmol) in 1.5 ml hexane was added over a period of 1 minute and the mixture stirred at −78°C for an additional minute. The mixture at this time was almost clear. Compound 13(b) (750 mg, 2.1 mmol) in 1.5 ml of hexane was added over a period of 1 minute resulting in a clear yellow solution which was stirred at −78°C for an additional 15 minutes. The reaction mixture was then flash chromatographed on 100 grams of silica gel eluted with 15% ethyl acetate in hexane to give compound 13(c).

(d) 3-Hydroxy-4-[(2-carboxyethyl)-thio]-5(Z)-octadecenoic acid

Compound 13(c) (0.2 g, 0.46 mmol) was dissolved in 5 ml of methanol and stirred under an argon atmosphere at 0°C. A 1*N* solution of sodium hydroxide (2 ml, 2 mmol) was added dropwise over a period of 0.5 minute. The ice bath was removed and the reaction allowed to warm to room tmeperature for 2 hours. Most of the methanol was evaporated and the aqueous residue was cooled in an ice bath and acidified with dilute hydrochloric acid. The aqueous phase was extracted twice with diethyl ether. The combined ether extracts were dried over anhydrous sodium sulfate, filtered, and evaporated to give crude product. This was recrystallized from diethyl ether-hexane to give the desired compound, mp 88—97°C. Anal. Calcd. C: 62.65; H: 9.51; S: 7.96; Found C: 62.32; H: 9.38; and S: 8.10.

Example 14

Preparation of 5-[(2-carboxyethyl)thio]-6(Z)-nonadecenoic acid

(a) 2-Bromo-5-carbomethoxypentanoic acid 14(a)

To a solution of 30 gm (0.187 M) of 5-carbomethoxypentanoic acid in 250 ml of chloroform was added 90 gm of thionyl chloride. The mixture was refluxed for 2 hours, cooled and the solvent removed under vacuum. The residue was redissolved in 200 ml of carbon tetrachloride, 40 gm (0.225 M) of N-bromosuccinimide was added, the mixture heated to reflux, eight drops of a HBr/acetic acid mixture added, and the reaction refluxed for 3 hours. After cooling, the mixture was filtered and evaporated to dryness. The residue was dissolved in 250 ml of acetone to which was added dropwise 200 ml of a freshly prepared 5% sodium bicarbonate solution. After stirring for 15 minutes during which time the pH was constantly adjusted to pH 9 with sodium bicarbonate the mixture was acidified with dilute HCl, to a pH 1.0, concentrated in vacuo, and extracted with chloroform. The organic phase was extracted with dilute bicarbonate solution, acidified and reextracted with chloroform. The extract was dried over $MgSO_4$, filtered and evaporated to dryness to afford the desired compound as a viscous oil.

(b) 5-Carbomethoxy-2-[(methoxycarbonylpropyl)thio]pentanoic acid 14(b)

A mixture of 42 gm (0.175 M) of 14(a), 50.68 g (0.42 M) of methyl-3-mercaptopropionate and 147 ml (1.05 M) of triethylamine in 250 ml of methanol was refluxed for two hours, cooled and concentrated in vacuum. The residue was dissolved in ethyl acetate and extracted with 5% sodium bicarbonate. The aqueous extract was acidified with 3N HCl, extracted with chloroform, the extract dried over $MgSO_4$, filtered and evaporated to dryness to afford the desired compound.

(c) 5-Carbomethoxy-2[(methoxycarbonylpropyl)thio]-pentanol 14(c)

A solution of 14(b), (40 g, 0.142 M) in THF (200 ml) was placed in a methanol-ice (−10°C) bath. The contents were permitted to cool for 20 minutes at which time 12.57 g (0.156 M) of borane-methyl sulfide complex was added slowly over a period of 50 minutes, followed by refluxing for 30 minutes. Acetic acid (10 ml) was then added and excess THF evaporated. The reaction mixture was dissolved in ethyl acetate (300 ml), washed with 5% sodium bicarbonate, dried over $MgSO_4$ and filtered. Evaporation afforded the crude alcohol.

(d) 5-Carbomethoxy-2[(methoxycarbonylpropyl)thio]-pentanal 14(d)

Methylene chloride (10 ml) and $Me_2SO$ (1.78 g, 0.23 M) were combined and cooled to −65°C. TFAA (1.73 g, 0.015 M) was added dropwise to the cold solution, at which time a white precipitate formed. After 5 minutes at −65°C, a solution of 14(c) (2.0 g, 7.58 mmol) in $CH_2Cl_2$ (5 ml) was added dropwise while maintaining the reaction mixture at −65°C. The mixture was then stirred at −65°C for 30 minutes, followed by addition of TEA (2.30 g, 0.023 M) dropwise. A temperature below −60°C was maintained until addition of TEA was complete. The bath was then removed and the reaction permitted to warm to room temperature. The mixture was diluted with $CH_2Cl_2$, washed with 3N HCl, $H_2O$, twice with 5% $NaHCO_3$ and once with brine. The organic phase was then dried over $MgSO_4$, filtered and evaporated to dryness to afford the crude aldehyde.

(e) Methyl-5-[(2-carbomethoxyethyl)thio]-6(Z)-non-adecanoate 14(e)

A mixture of tridecyltriphenyl phosphonium bromide (1.76 g, 3.35 M) and THF (20 ml) was permitted to stir for 5 minutes at room temperature. The solution was then cooled to −68°C and stirred for an additional

10 minutes. At which time n-butyllithium (3.05 mmol) was slowly added while maintaining a −65°C temperature. Following stirring for 10 minutes at −68°C the reaction mixture was stirred for an additional 10 minutes at −10°C. Upon recooling to −68°C, (0.918 g, 3.50 mmol) of 14(d) was dissolved in THF (10 ml) and slowly added. The reaction was stirred for one hour at −68°C followed by removal of the bath in order to warm the mixture to room temperature. THF was then evaporated and the residue dissolved in ethyl acetate, which was washed with 3N HCl, water and twice with 5% $NaHCO_3$. The extract was dried over $MgSO_4$, filtered and evaporated to dryness. The product was flash chromatographed with 91% hexane: 9% EtOAc to provide the nonadecanoate.

(f) 5-[(2-Carboxyethyl)thio]-6(Z)-nonadecanoic acid

Potassium carbonate (0.81 g, 5.84 mmol) was dissolved in $H_2O$ (10 ml), to which was added methanol (5 ml). The solution was stirred for 2 minutes at room temperature, followed by the addition of 14(e) (100 mg; 0.234 mmol) in MeOH (24 ml). The mixture was permitted to stir overnight at room temperature, after which time excess MeOH was evaporated and the remaining aqueous phase was then washed with ethyl acetate, acidified to a pH=1.0 with 3N HCl and extracted twice with ethyl acetate, the extract was dried over $MgSO_4$, filtered and evaporated to an oil. The product was recrystallized from ether-petroleum ether and cooled for one hour to afford the nonadecanoic acid as a white crystalline solid (mp 53—54°C).

Example 15

Preparation of 5-[(2-carboxyethyl)thio]-4-hydroxy-6(Z)-nonadecenamide

The above named compound was prepared by treatment of the compound of Example 5 with 1% solution of trifluoroacetic anhydride in $CH_2Cl_2$ for 2 hours at room temperature, followed by treatment with $NH_3$ in methanol solution at 0°C for 0.5 hour and room temperature overnight. Product was obtained as a crystalline solid mp 82—85°C; Anal. Calcd. for $C_{22}H_{41}NO_4S \cdot 0.3\ M\ NH_3$: C, 62.77; H, 9.89; N, 4.33. Found: C, 62.67, 62.98; H, 9.92, 9.78; N, 3.98, 4.00.

Example 16

Preparation of 5-[(3-carboxymethylamino-3-oxopropyl)thio]-4-hydroxy-6(Z)-nonadecanamide

A mixture of 0.9 g (1.4 mmol) of compounds of Example 4(g) in 100 ml of 3.8% anhydrous ammonia in ethanol solution was stirred at room temperature for 2 days. The reaction mixture was concentrated to dryness. The residue was azeotroped with methylene chloride to give 0.9 g of off-white powder. The hydroscopic material was dissolved in 10% NaOH solution (5 ml) and chromatographed on a 3-cm-by-9-cm column of XAD-7 resin. After washing with $H_2O$ (100 ml), the column was eluted with aqueous methanol solution (1:1) taking 20 ml per fraction. Fraction 13 was collected to give the desired product as a white amorphous powder, mp. 195—8°C. Anal. Calcd. for $C_{24}H_{44}N_3O_5S \cdot Na(H)\ 2H_2O$: C, 52.82; H, 8.87; N, 7.70. Found: C, 52.65; H, 8.15; N, 7.72.

Example 17

Preparation of 4-hydroxy-5-[(2-carboxyethyl)thio]-(Z)-heptadecenoic acid

The above named compound was prepared by the general method of Example 5 using 4,5-epoxy-6(Z)-heptadecenoic acid methyl ester, which was obtained by employing the general method of Example 4(e) using undecyltriphenyl phosphonium bromide. The product obtained had a melting point of 78.5—80°C. Anal. calcd.: C, 61.82; H, 9.34; S, 8.25. Found: C, 61.82; H, 9.10; S, 8.42.

Example 18

Preparation of 4-Hydroxy-5-[(2-carboxyethyl)thio]-6(Z)-heneicosenoic acid

The above named compound was prepared by the general method of Example 5 using 4,5-epoxy-6(Z)-heneicosenoic acid methyl ester, which was obtained by employing the general method of Example 4(e) using pentadecyltriphenyl phosphonium bromide. The product obtained had a melting point 59—61°C. Anal. calcd.: C, 64.82; H, 9.97; S, 7.21. Found: C, 65.16; H, 10.07; S, 7.36.

Example 19

Preparation of 4-[(2-carboxyethyl)thio]-5(Z)-octadecenoic acid

(a) Methyl-4-bromo-4-carboxybutanoate 19(a)

Methyl-4-(chloroformyl)butyrate (30.0 g, 0.182 M) was charged to a flask, along with 200 ml of $CCl_4$. To this solution N-bromosuccinimide (40.0 g, 0.219 M) was added. The reaction mixture was heated to reflux and seven drops of 47% aqueous HBr was added, reflux conditions continued for approximately 2 hours. Following cooling to room temperature, the reaction was filtered and evaporated to dryness. The residue was then placed in the refrigerator overnight. The residue was dissolved in 250 ml of acetone, to which is added saturated sodium bicarbonate during which time the pH was adjusted to nine. With dilute HCl the mixture was then acidified to a pH=1.0, concentrated and extracted with chloroform. The extract was dried over $MgSO_4$, filtered and evaporated to dryness to afford the desired compound as an oil.

(b) Methyl-4-[(2-carbomethoxyethyl)thio]-4-carboxy butanoate 19(b)

To a stirred solution of 29.0 g (0.24 M) of methyl-3-mercaptopropionate and 61.0 g (0.60 M) of

triethylamine in 200 ml of methanol was added 22.5 (0.10 M) of 19(a) under argon. After stirring at room temperature for ten minutes, the reaction was heated to reflux for one and a half hours, cooled to room temperature and concentrated in a vacuum. The residue was then diluted with ethyl acetate, extracted with 5% sodium bicarbonate, acidified to pH 1.6 with dilute HCl and reextracted into chloroform several times. Organic extracts were then combined, dried over $MgSO_4$, filtered and evaporated to dryness to afford the desired compound.

(c) Methyl-4-[(2-carbomethoxyethyl)thio]-5-hydroxy pentanoate 19(c)

A solution of 19(b) (21.4 g; 0.08 M) in THF (175 ml) was cooled to −10°C in a methanol-ice bath. The contents were permitted to cool for 10 minutes at which time borane methyl sulfide complex (7.2 g; 0.09 M) was added slowly. Following addition reaction was warmed to room temperature and then refluxed for approximately 15 minutes. After cooling acetic acid (6 ml) was added and excess THF evaporated. The reaction mixture was dissolved in ethyl acetate, washed with 5% sodium bicarbonate, dried over $MgSO_4$, filtered, evaporated to dryness to afford the crude alcohol. This material was then flash chromatographed using a 60% ethyl acetate in hexane system to yield a purer product.

(d) Methyl-4-[(2-carbomethoxymethyl)thio]-4-formyl butanoate 19(d)

Methylene chloride (20 ml) and $Me_2SO$ (1.14 ml; 0.016 M) were combined and cooled to −65°C. TFAA (0.91 ml; 0.012 M) was added dropwise to the cold solution at which time a white precipitate formed. After 5 minutes a solution of 19(c) (2.0 g; 0.008 M) in methylene chloride (3 ml) was added dropwise while maintaining the temperature at −65°C. The reaction mixture was then stirred for thirty minutes followed by addition of TEA (3.1 ml; 0.022 M) dropwise. The reaction mixture was stirred for an additional thirty minutes at −65°C, at which it was diluted with methylene chloride, washed with dilute HCl, water and then twice with 5% sodium bicarbonate. The organic phase was then dried over $MgSO_4$, filtered and evaporated to dryness to afford the crude aldehyde. This material was flash chromatographed using 50% ethyl acetate in hexane resulting in purer product.

(e) Methyl-4-[(2-carbomethoxyethyl)thio]-5(Z)-octadecenoate 19(e)

A mixture of tridecyltriphenyl phosphonium bromide (1.27 g; 2.4 mmol) and THF (20 ml) was allowed to stir for 3 minutes at room temperature. The solution was then cooled to −68°C, at which time n-butyllithium (1.05 ml; 2.4 mmol) was added slowly while maintaining a reaction temperature less than −65°C. Following stirring at −68°C the reaction was permitted to warm to −10°C for approximately 10 minutes. Upon recooling to −68°C, (0.05 g; 2.0 mmol) of 19(d) was dissolved in THF (10 ml) and slowly added. The reaction was stirred for one hour at −68°C followed by removal of the bath. Product was then triturated into hexane and also into ether, hexane and ether fractions were combined, evaporated to dryness and the remaining residue was flash chromatographed affording the desired octadecenoate.

(f) 4-[(2-Carboxyethyl)thio]-5(Z)-octadecenoic acid

(319 mg; 0.826 mmol) of 19(e) was dissolved in methanol (7.5 ml) and permitted to stir under argon at 0°C. A 1N solution of sodium hydroxide (3.1 ml; 3.08 mmol) was added dropwise. The ice bath was then removed and the reaction allowed to warm to room temperature for approximately three hours. After which it was placed in the refrigerator and stored overnight. Most of the methanol was then evaporated and the aqueous residue was cooled in an ice bath followed by acidification with dilute HCl. The aqueous phase was extracted twice with diethyl ether. The ether extracts were then combined and dried over magnesiums sulfate, filtered and evaporated to give crude product. This was then recrystallized from diethyl ether-hexane to afford the final product, mp 81°C; Anal. Calcd. C: 65.24; H: 9.91; S: 8.29; Found C: 65.50; H: 10.05; S: 8.61.

Example 20

As a specific embodiment of a composition of this invention, an active ingredient, such as the compound of Example 4(i), is dissolved in sterile water at a concentration of 0.5 percent and aerosolized from a nebulizer operating at an air flow adjusted to deliver the desired aerosolized weight of drug.

Example 21

As an additional specific embodiment of a composition of this invention, an active ingredient, such as the compound of Example 5, is admixed with mannitol at a concentration of 1.0 percent and administered from a powder inhalation device adjusted to deliver the desired weight of drug.

**Claims for the Contracting States: BE, CH, DE, FR, GB, IT, LI, LU, NL, SE**

1. A compound represented by the formula (I)

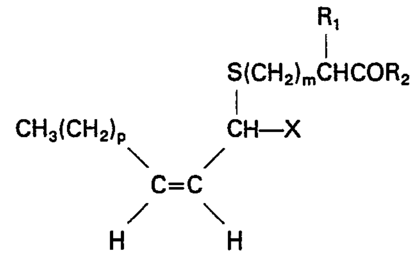

(I)

wherein m is 0, 1 or 2; p is 9, 10, 11, 12 or 13; $R_1$ is hydrogen, amino or

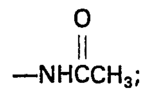;

$R_2$ is hydroxyl, amino, $-NHCH_2CO_2H$,

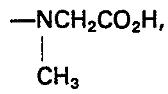, 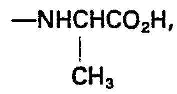, 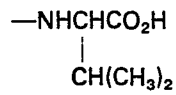

or $-NHCH_2CONH_2$; and X is $-CO_2H$, $-CH_2OH$,

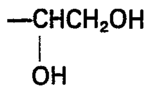 or 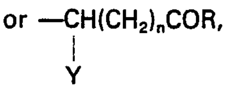,

wherein n is 1 or 2, Y is hydrogen or hydroxyl and R is hydroxyl or amino with the proviso that when m is 0, $R_1$ is hydrogen, or a pharmaceutically acceptable salt thereof.

2. A compound of Claim 1 wherein X is $-CO_2H$, $-CH_2OH$ or

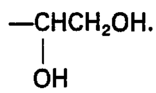.

3. A compound of Claim 2 which is
2-[(2-carboxyethyl)thio]-3(Z)-hexadecenoic acid,
2-[(2-carboxyethyl)thio]-3(Z)-hexadecen-1-ol or
3-[(2-carboxyethyl)thio]-4(Z)-heptadecen-1,2-diol.

4. A compound of Claim 1 wherein X is

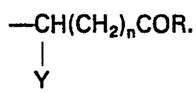.

5. A compound of Claim 4 wherein R is hydroxyl and Y is hydroxyl.

6. A compound of Claim 5 which is
4-hydroxy-5[(2-carboxyethyl)thio]-6(Z)-nonadecenoic acid,
5[(3-carboxymethylamino-3-oxopropyl)thio]-4-hydroxy-6(Z)-nonadecenoic acid,
5[(3-carboxymethyl-N-methylamino-3-oxopropyl)thio]-4-hydroxy-6(Z)-nonadecenoic acid,
5[(2-amino-3-carboxamidomethylamino-3-oxo-propyl)thio]-4-hydroxy-6(Z)-nonadecenoic acid,
4-hydroxy-5-[(carboxymethyl)thio]-6(Z)-nonadecenoic acid,
5-[[(2-aminocarbonyl)ethyl]thio]-4-hydroxy-6(Z)-nonadecenoic acid,
4-hydroxy-5-[(2-amino-2-carboxyethyl)thio]-6(Z)-nonadecenoic acid,
4(R)-hydroxy-5(S)-[(2-amino-2-carboxyethyl)thio]-6(Z)-nonadecenoic acid,
4(S)-hydroxy-5(R)-[(2-amino-2-carboxyethyl)thio]-6(Z)-nonadecenoic acid, or
4-hydroxy-5-[(3-carboxypropyl)thio-6(Z)-nonadecenoic acid.

7. A compound of Claim 5 which is
5[(2-amino-3-carboxymethylamino-3-oxopropyl)thio]-4-hydroxy-6(Z)-nonadecenoic acid,
5(S)-[(2-amino-3-carboxymethylamino-3-oxopropyl)thio]-4(R)-hydroxy-6(Z)-nonadecenoic acid, or
5(R)-[(2-amino-3-carboxymethylamino-3-oxopropyl)thio]-4(S)-hydroxy-6(Z)-nonadecenoic acid.

8. A compound of Claim 5 which is
3-hydroxy-4-[(2-carboxyethyl)thio]-5(Z)-octadecenoic acid,
4-hydroxy-5-[(2-carboxyethyl)thio]-6(Z)-heptadecenoic acid, or
4-hydroxy-5[(2-carboxyethyl)thio]-6(Z)-heneicosenoic acid.

9. A compound of Claim 4 which is
4[(2-carboxyethyl)thio]-5(Z)-octadecenoic acid,
5[(2-carboxyethyl)thio]-6(Z)-nonadecenoic acid,
5[(3-carboxymethylamino-3-oxopropyl)thio]-4-hydroxy-6(Z)-nonadecenamide, or
4-hydroxy-5-[(2-carboxyethyl)thio]-6(Z)-nonadecenamide.

10. A pharmaceutical composition for inhibiting the effects of leukortriene in subjects in need of such inhibition comprising a pharmaceutical carrier or diluent and a nontoxic amount sufficient to produce said inhibition of a compound of Claim 1.

11. A compound of the formula (C)

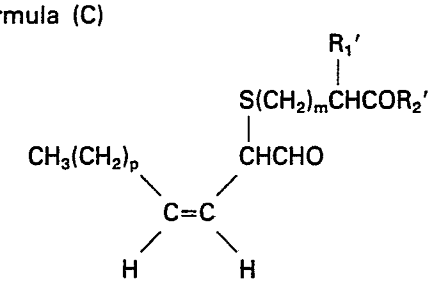

(C)

wherein m is 0, 1 or 2; p is 9, 10, 11, 12 or 13; $R_1'$ is hydrogen,

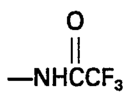 or 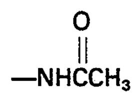

and $R_2'$ is amino, $—NHCH_2CO_2R_3'$,

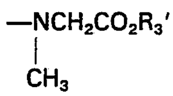 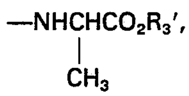, 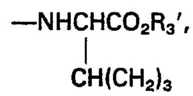,

$—NHCH_2CONH_2$ or $OR_3'$ wherein $R_3'$ is an alkyl radical containing one to six carbon atoms with the proviso that when m is 0, $R_1'$ is hydrogen.

12. A process for preparing the compounds represented by the formula (I)

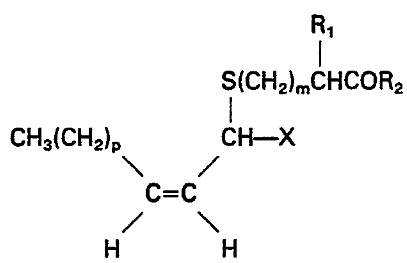

(I)

wherein m is 0, 1 or 2; p is 9, 10, 11, 12 or 13; $R_1$ is hydrogen, amino or

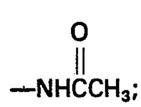

$R_2$ is hydroxyl, amino, $—NHCH_2CO_2H$,

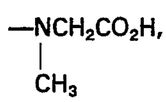, 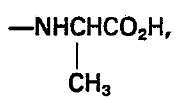, 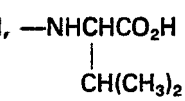

or $—NHCH_2CO\ NH_2$; and X is $—CO_2H$, $—CH_2OH$,

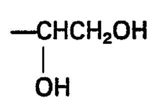 or 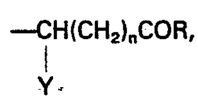

wherein n is 1 or 2, Y is hydrogen or hydroxyl and R is hydroxyl or amino with the proviso that when m is 0, $R_1$ is hydrogen, or a pharmaceutically acceptable salt thereof which comprises:

(A) when X is

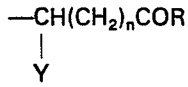

wherein Y is hydroxyl and n is 2, reacting

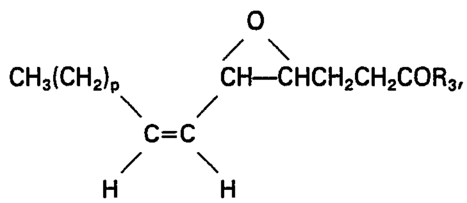

wherein $R_3$ is R or a radical easily convertible into R with

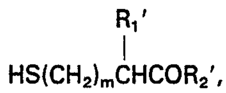

wherein $R_1'$ and $R_2'$ are respectively $R_1$ and $R_2$ or a radical easily convertible into $R_1$ and $R_2$;

(B) when X is

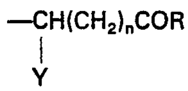

wherein Y is hydroxyl and n is 1, reacting

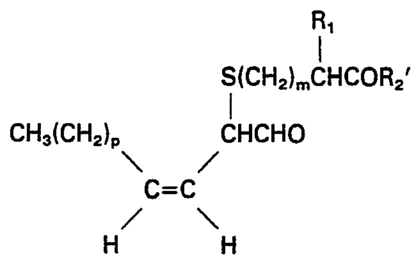

with $CH_3COR_3$;

(C) when X is

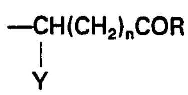

wherein Y is hydrogen, reacting

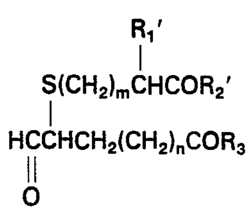

with $CH_3(CH_2)_pCH{=}P\phi 3$; where $\phi$ is phenyl

(D) when X is —$CO_2H$, reacting

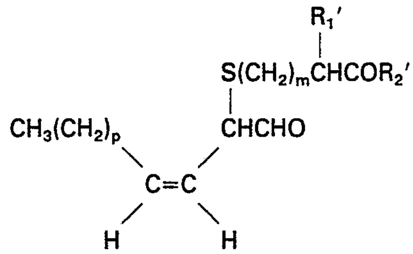

with an oxidizing agent;

(E) when X is —$CH_2OH$, reacting

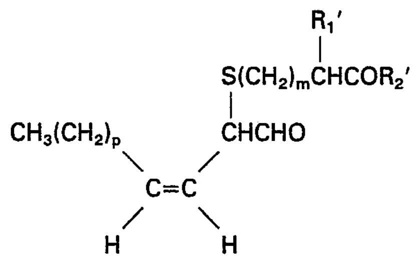

with a reducing agent; or

(F) when X is

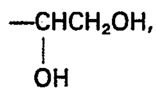

reacting

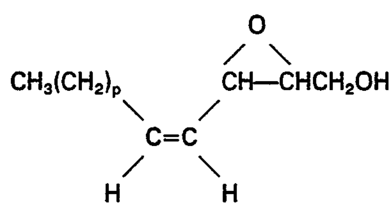

with

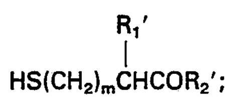

and then optionally converting $R_1'$, $R_2'$ and $R_3$ into $R_1$, $R_2$ and R and optionally forming the pharmaceutically acceptable salt of the compounds.

13. A compound of formula (I) as claimed in any of Claims 1 to 9 for use as an active therapeutic substance.

14. A compound of formula (I) as claimed in any of Claims 1 to 9 for use as an anti-asthmatic agent.

**Claims for the Contracting State AT:**

1. A process for preparing the compounds represented by the formula (I)

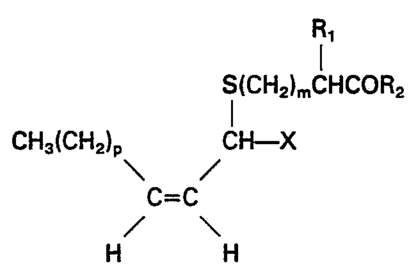

(I)

wherein m is 0, 1 or 2; p is 9, 10, 11, 12 or 13; $R_1$ is hydrogen, amino or

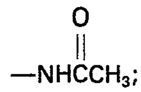

$R_2$ is hydroxyl, amino, $—NHCH_2CO_2H$,

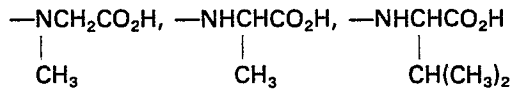

or $—NHCH_2CO\ NH_2$; and X is $—CO_2H$, $—CH_2OH$,

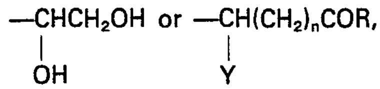

wherein n is 1 or 2, Y is hydrogen or hydroxyl and R is hydroxyl or amino with the proviso that when m is 0, $R_1$ is hydrogen, or a pharmaceutically acceptable salt thereof which comprises:

(A) when X is

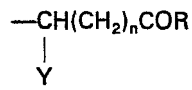

wherein Y is hydroxyl and n is 2, reacting

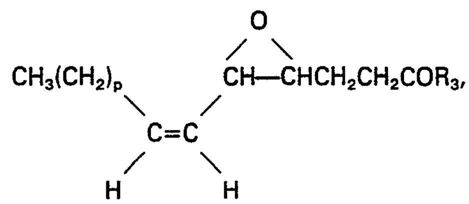

wherein $R_3$ is R or a radical easily convertible into R with

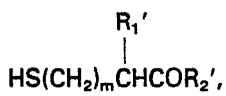

wherein $R_1'$ and $R_2'$ are respectively $R_1$ and $R_2$ or a radical easily convertible into $R_1$ and $R_2$;

(B) when X is

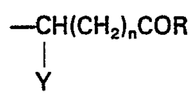

wherein Y is hydroxyl and n is 1, reacting

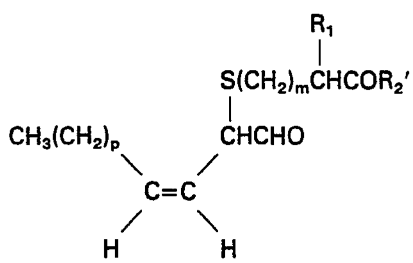

with $CH_3COR_3$;

(C) when X is

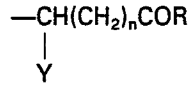

wherein Y is hydrogen, reacting

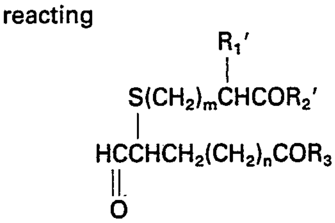

with $CH_3(CH_2)_pCH{=}P\phi_3$ where $\phi$ is phenyl;

(D) when X is $-CO_2H$, reacting

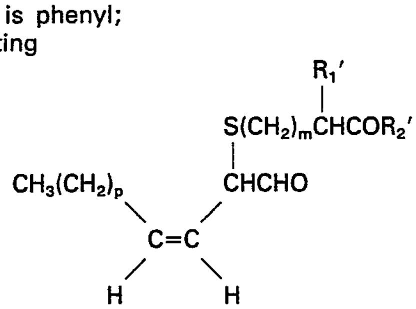

with an oxidizing agent;

(E) when X is $-CH_2OH$, reacting

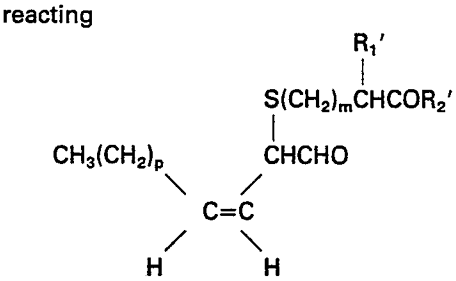

with a reducing agent; or

(F) when X is

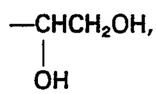

reacting

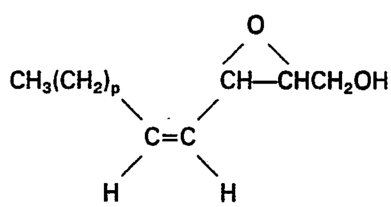

with

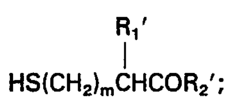

and then optionally converting $R_1'$, $R_2'$ and $R_3$ into $R_1$, $R_2$ and R and optionally forming the pharmaceutically acceptable salt of the compounds.

2. A process according to Claim 1 for preparing the compounds of the formula (III)

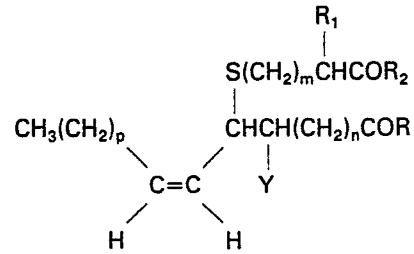

(III)

wherein n is 2 and Y is hydroxyl, which comprises reacting

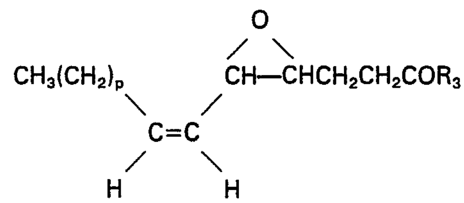

with

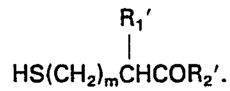

3. A process according to Claim 2 for preparing 5-[(2-amino-3-carboxymethylamino-3-oxopropyl)thio]-4-hydroxy-6(Z)-nonadecenoic acid which comprises reacting methyl 4,5-epoxy-6(Z)-nonadecenoate with 2-trifluoroacetamido-3-carbomethoxymethylamino-3-oxopropylmercaptan and converting the resultant compound with aqueous base to the desired product.

4. A process according to Claim 2 for preparing 4-hydroxy-5-[(2-carboxyethyl)thio]-6(Z)-nonadecenoic acid which comprises reacting methyl 4,5-epoxy-6(Z)-nonadecenoate with methyl-3-mercaptopropionate and converting the resultant compound with aqueous base to the desired product.

5. A process according to Claim 1 for preparing the compounds of the formula (III)

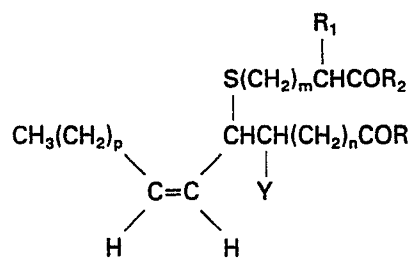

(III)

wherein n is 1 and Y is hydroxyl which comprises reacting

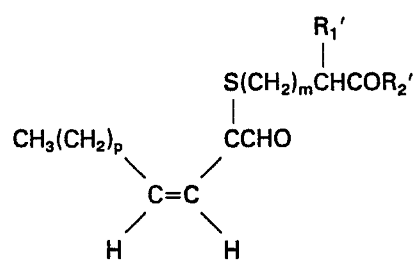

with $CH_3COR_3$.

6. A process according to Claim 5 for preparing 3-hydroxy-4-[(2-carboxyethyl)thio]-5(Z)-octadecenoic acid which comprises reacting 2-[(2-carboxyethyl)thio]-hexadec-3(Z)-enal with methyl acetate and converting the resultant compound with aqueous base to the desired product.

7. A process according to Claim 1 for preparing the compounds of the formula (III)

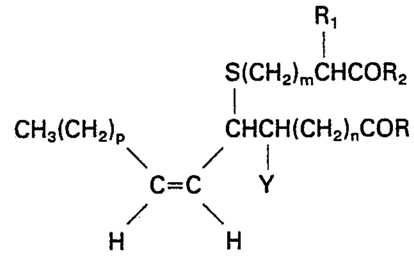

(III)

wherein Y is hydrogen which comprises reacting

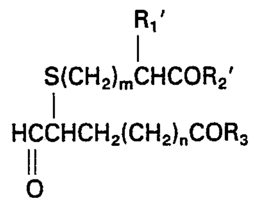

with $CH_3(CH_2)_pCH{=}P\phi_3$, where $\phi$ is phenyl.

8. A process according to Claim 7 for preparing 4-[(2-carboxyethyl)thio]-5(Z)-octadecenoic acid which comprises reacting methyl-4-[(2-carbomethoxyethyl)thio]-4-formylbutanoate with tridecyltriphenylphosphonium ylid and converting the resulting compound in aqueous base to the desired product.

9. A process for the preparation of a pharmaceutical composition which comprises combining a compound of formula (I) as defined in any of Claims 1 to 8 with a pharmaceutically acceptable carrier therefor.

**Patentansprüche für die Vertragsstaaten: BE, CH, DE, FR, GB, IT, LI, LU, NL, SE**

1. Eine Verbindung der Formel (I)

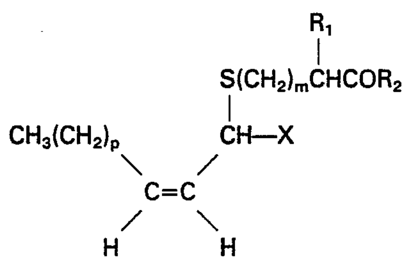

(I)

in der m den Wert 0, 1 oder 2, p den Wert 9, 10, 11, 12 oder 13 hat, $R_1$ ein Wasserstoffatom, eine Aminogruppe oder eine Gruppe der Formel

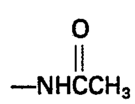

besitzt, $R_2$ eine Hydroxyl- oder Aminogruppe oder eine Gruppe der Formeln $-NHCH_2CO_2H$,

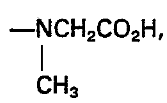, 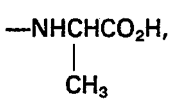, 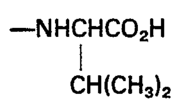

oder $-NHCH_2CONH_2$ darstellt, und X eine Gruppe der Formeln $-CO_2H$, $-CH_2OH$,

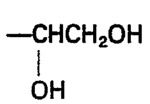 oder 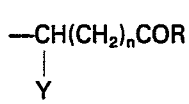

bedeutet, wobei n den Wert 1 oder 2 hat, Y ein Wasserstoffatom oder eine Hydroxylgruppe darstellt und R eine Hydroxyl- oder Aminogruppe bedeutet, mit der Maßgabe, daß $R_1$ ein Wasserstoffatom bedeutet, wenn m den Wert 0 hat, oder ein pharmazeutisch verträgliches Salz davon.

2. Verbindung nach Anspruch 1, in der X eine Gruppe der Formeln $—CO_2H$, $—CH_2OH$ oder

$—CHCH_2OH$
|
OH

bedeutet.

3. Verbindung nach Anspruch 2, nämlich
2-[(2-Carboxyäthyl)-thio]-3(Z)-hexadecensäure,
2-[(2-Carboxyäthyl)-thio]-3(Z)-hexadecen-1-ol oder
3-[(2-Carboxyäthyl)-thiol]-4(Z)-heptadecen-1,2-diol.

4. Verbindung nach Anspruch 1, in der X eine Gruppe der Formel

$—CH(CH_2)_nCOR$
|
Y

darstellt.

5. Verbindung nach Anspruch 4, in der R eine Hydroxylgruppe und Y eine Hydroxylgruppe bedeuten.

6. Verbindung nach Anspruch 5, nämlich
4-Hydroxy-5[(2-carboxyäthyl)-thio]-6(Z)-nonadecensäure,
5-[(3-Carboxymethylamino-3-oxopropyl)-thio]-4-hydroxy-6(Z)-nonadecensäure,
5[(3-Carboxymethyl-N-methylamino-3-oxopropyl)-thio]-4-hydroxy-6(Z)-nonadecensäure,
5[(2-Amino-3-carboxamidomethylamino-3-oxopropyl)-thio]-4-hydroxy-6(Z)-nonadecensäure,
4-Hydroxy-5-[(carboxymethyl)-thio]-6(Z)-nonadecensäure,
5-[[(2-Aminocarbonyl)-äthyl]-thio]-4-hydroxy-6(Z)-nonadecensäure,
4-Hydroxy-5-[(2-amino-2-carboxyäthyl)-thio]-6(Z)-nonadecensäure,
4(R)-Hydroxy-5(S)-[(2-amino-2-carboxyäthyl)-thio]-6(Z)-nonadecensäure,
4(S)-Hydroxy-5(R)-[(2-amino-2-carboxyäthyl)-thio]-6(Z)-nonadecensäure oder
4-Hydroxy-5-[(3-carboxypropyl)-thio]-6(Z)-nonadecensäure.

7. Verbindung nach Anspruch 5, nämlich
5[(2-Amino-3-carboxymethylamino-3-oxopropyl)-thio]-4-hydroxy-6(Z)-nonadecensäure,
5(S)-[(2-Amino-3-carboxymethylamino-3-oxopropyl)-thio]-4(R)-hydroxy-6(Z)-nonadecensäure oder
5(R)-[(2-Amino-3-carboxymethylamino-3-oxopropyl)-thio]-4(S)-hydroxy-6(Z)-nonadecensäure.

8. Verbindung nach Anspruch 5, nämlich
3-Hydroxy-4-[(2-carboxyäthyl)-thio]-5(Z)-octadecensäure,
4-Hydroxy-5-[(2-carboxyäthyl)-thio]-6(Z)-heptadecensäure oder
4-Hydroxy-5[(2-carboxyäthyl)-thio]-6(Z)-heneicosensäure.

9. Verbindung nach Anspruch 4,
4-[(2-Carboxyäthyl)-thio]-5(Z)-octadecensäure,
5-[(2-Carboxyäthyl)-thio]-6(Z)-nonadecensäure,
5-[(3-Carboxymethylamino-3-oxopropyl)-thio]-4-hydroxy-6(Z)-nonadecensäure, oder
4-Hydroxy-5-[(2-carboxyäthyl)-thio]-6(Z)-nonadecenamid.

10. Arzneimittel zur Inhibierung der Wirkungen von Leukotrien in Patienten, die eine solche Inhibierung benötigen, umfassend einen pharmazeutischen Träger oder ein Verdünnungsmittel und eine nichttoxische Menge einer Verbindung nach Anspruch 1, die zur Erzeugung der Inhibierung ausreicht.

11. Verbindung der Formel (C)

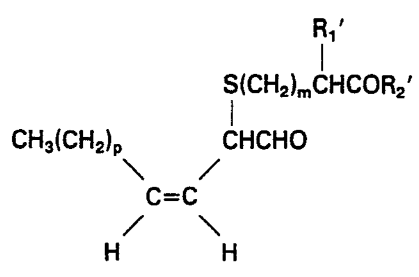

(C)

in der m den Wert 0, 1 oder 2 hat, p den Wert 9, 10, 11, 12 oder 13 hat, $R_1'$ ein Wasserstoffatom oder eine Gruppe der Formeln

O || —NHCCF$_3$ oder O || —NHCCH$_3$

bedeutet, und $R_2''$ eine Aminogruppe oder eine Gruppe der Formeln $—NHCH_2CO_2R_3'$,

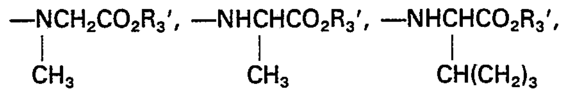

—$NHCH_2CONH_2$ oder $OR_3'$ darstellt, wobei $R_3'$ einen Alkylrest mit 1 bis 6 Kohlenstoffatomen bedeutet, mit der Maßgabe, daß $R_1'$ ein Wasserstoffatom bedeutet, wenn m den Wert 0 hat.

12. Verfahren zur Herstellung der Verbindungen der Formel (I)

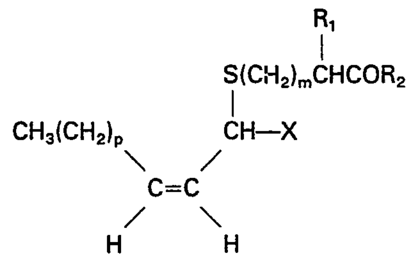

(I)

in der m den Wert 0, 1 oder 2, p den Wert 9, 10, 11, 12 oder 13 hat, $R_1$ ein Wasserstoffatom, eine Aminogruppe oder eine Gruppe der Formel

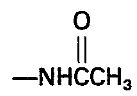

besitzt, $R_2$ eine Hydroxyl- oder Aminogruppe oder eine Gruppe der Formeln —$NHCH_2CO_2H$,

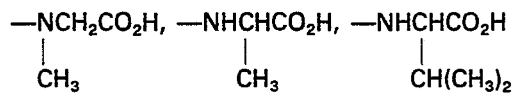

oder —$NHCH_2CONH_2$ darstellt, und X eine Gruppe der Formeln —$CO_2H$, —$CH_2OH$,

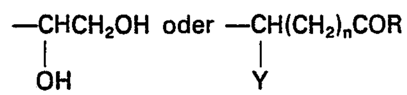

bedeutet, wobei n den Wert 1 oder 2 hat, Y ein Wasserstoffatom oder eine Hydroxylgruppe darstellt und R eine Hydroxyl- oder Aminogruppe bedeutet, mit der Maßgabe, daß $R_1$ ein Wasserstoffatom bedeutet, wenn m den Wert 0 hat, oder eines pharmazeutisch verträglichen Salzes davon, das umfaßt:

(A) wenn X einen Rest der Formel

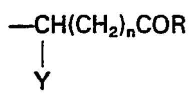

bedeutet, in der Y eine Hydroxylgruppe darstellt und n den Wert 2 hat, Umsetzung einer Verbindung der Formel

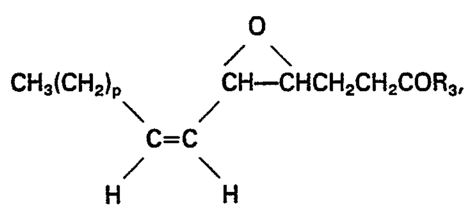

in der $R_3$ die Bedeutung von R hat oder einen leicht in R umwandelbaren Rest darstellt, mit einer Verbindung der Formel

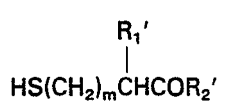

in der $R_1'$ und $R_2'$ jeweils die Bedeutung von $R_1$ und $R_2$ haben, oder einen leicht in $R_1$ und $R_2$ umwandelbaren Rest darstellen;

(B) wenn X eine Gruppe der Formel

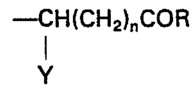

bedeutet, in der Y eine Hydroxylgruppe darstellt und n den Wert 1 hat, Umsetzung einer Verbindung der Formel

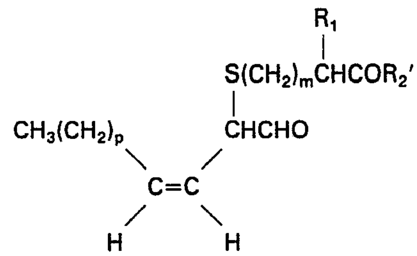

mit $CH_3COR_3$;

(C) wenn X eine Gruppe der Formel

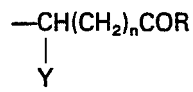

bedeutet, in der Y ein Wasserstoffatom darstellt, Umsetzung einer Verbindung der Formel

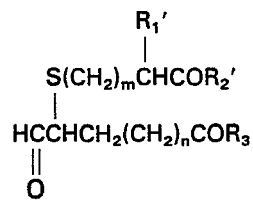

mit einer Verbindung der Formel $CH_3(CH_2)_pCH{=}P\phi_3$, in der $\phi$ eine Phenylgruppe bedeutet;

(D) wenn X eine Gruppe der Formel $—CO_2H$ bedeutet, Umsetzung einer Verbindung der Formel

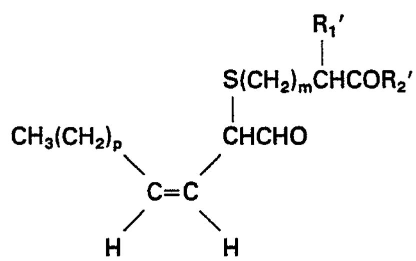

mit einem Oxidationsmittel;

(E) wenn X eine Gruppe der Form $CH_2OH$ darstellt, Umsetzung einer Verbindung der Formel

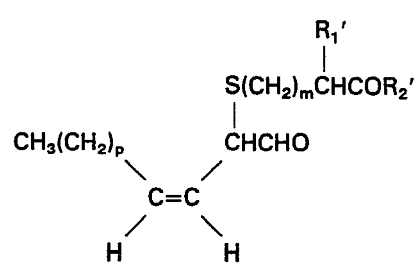

mit einem Reduktionsmittel; oder

(F) wenn X eine Gruppe der Formel

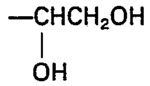

bedeutet, Umsetzung einer Verbindung der Formel

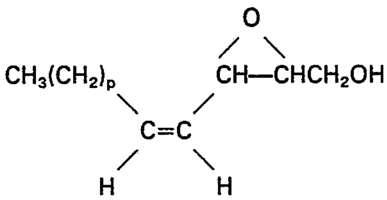

mit einer Verbindung der Formel

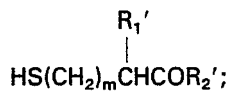

anschließend gegebenenfalls Umwandlung der Reste $R_1'$, $R_2'$ und $R_3$ in die Reste $R_1$, $R_2$ und R, und gegebenenfalls Erzeugung eines pharmazeutisch verträglichen Salzes der Verbindungen.

13. Eine Verbindung der Formel (I) nach einem der Ansprüche 1 bis 9 zur Verwendung als therapeutischer Wirkstoff.

14. Eine Verbindung der Formel (I) nach einem der Ansprüche 1 bis 9 zur Verwendung als Wirkstoff gegen Asthma.

**Patentansprüche für den Vertragsstaat: AT**

1. Verfahren zur Herstellung der Verbindungen der Formel (I)

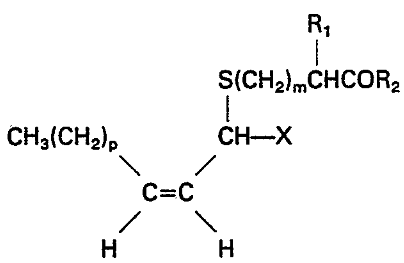

(I)

in der m den Wert 0, 1 oder 2, p den Wert 9, 10, 11, 12 oder 13 hat, $R_1$ ein Wasserstoffatom, eine Aminogruppe oder eine Gruppe der Formel

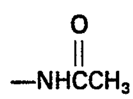

besitzt, $R_2$ eine Hydroxyl- oder Aminogruppe oder eine Gruppe der Formeln $—NHCH_2CO_2H$,

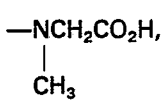, 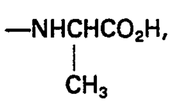, 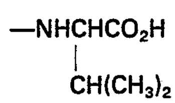

oder $—NHCH_2CONH_2$ darstellt, und X eine Gruppe der Formeln $—CO_2H$, $—CH_2OH$,

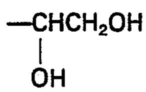 oder 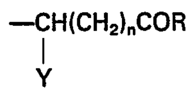

bedeutet, wobei n den Wert 1 oder 2 hat, Y ein Wasserstoffatom oder eine Hydroxylgruppe darstellt und R eine Hydroxyl- oder Aminogruppe bedeutet, mit der Maßgabe, daß $R_1$ ein Wasserstoffatom bedeutet, wenn m den Wert 0 hat, oder eines pharmazeutisch verträglichen Salzes davon, das umfaßt:

(A) wenn X einen Rest der Formel

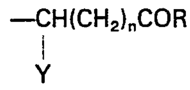

bedeutet, in der Y eine Hydroxylgruppe darstellt und n den Wert 2 hat, Umsetzung einer Verbindung der Formel

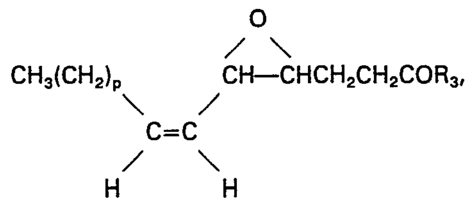

in der $R_3$ die Bedeutung von R hat oder einen leicht in R umwandelbaren Rest darstellt, mit einer Verbindung der Formel

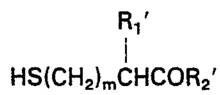

in der $R_1'$ und $R_2'$ jeweils die Bedeutung von $R_1$ und $R_2$ haben, oder einen leicht in $R_1$ und $R_2$ umwandelbaren Rest darstellen;

(B) wenn X eine Gruppe der Formel

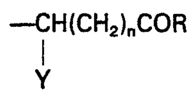

bedeutet, in der Y eine Hydroxylgruppe darstellt und n den Wert 1 hat, Umsetzung einer Verbindung der Formel

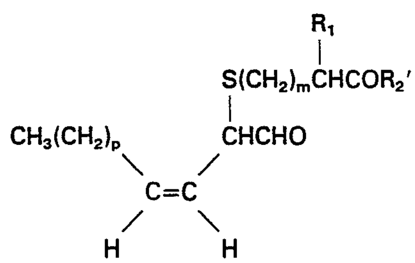

mit $CH_3COR_3$;

(C) wenn X eine Gruppe der Formel

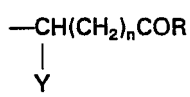

bedeutet, in der Y ein Wasserstoffatom darstellt, Umsetzung einer Verbindung der Formel

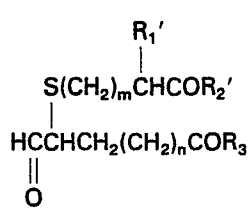

mit einer Verbindung der Formel $CH_3(CH_2)_pCH{=}P\phi_3$ in der $\phi$ eine Phenylgruppe bedeutet;

(D) wenn X eine Gruppe der Formel $—CO_2H$ bedeutet, Umsetzung einer Verbindung der Formel

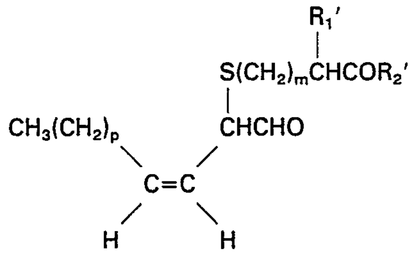

mit einem Oxidationsmittel;

(E) wenn X eine Gruppe der Form $CH_2OH$ darstellt, Umsetzung einer Verbindung der Formel

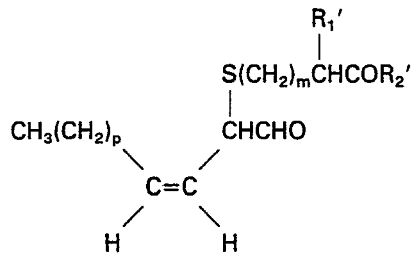

mit einem Reduktionsmittel; oder

(F) wenn X eine Gruppe der Formel

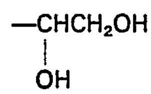

bedeutet, Umsetzung einer Verbindung der Formel

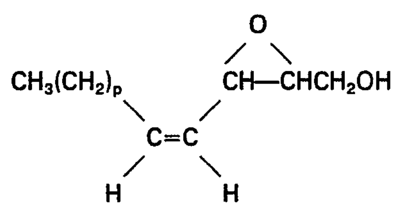

mit einer Verbindung der Formel

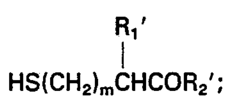

anschließend gegebenenfalls Umwandlung der Reste $R_1'$ $R_2'$ und $R_3$ in die Reste $R_1$, $R_2$ und R, und gegebenenfalls Erzeugung eines pharmazeutisch verträglichen Salzes der Verbindungen.

2. Verfahren nach Anspruch 1 zur Herstellung der Verbindungen der Formel (III)

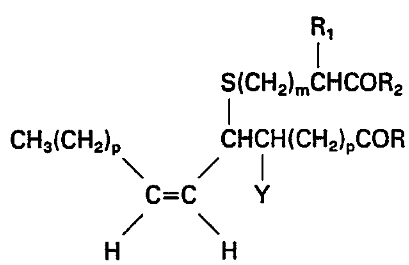
(III)

in der n den Wert 2 hat und Y eine Hydroxylgruppe ist, durch Umsetzung einer Verbindung der Formel

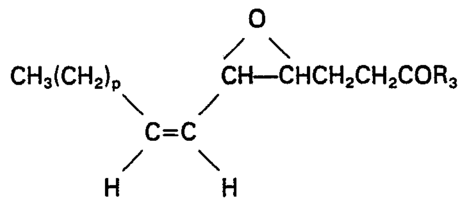

mit einer Verbindung der Formel

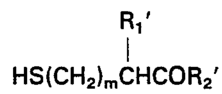

3. Verfahren nach Anspruch 2 zur Herstellung von 5-[(2-Amino-3-carboxymethylamino-3-oxopropyl)-thio]-4-hydroxy-6(Z)-nonadecensäure durch Umsetzung von 4,5-Epoxy-6(Z)-nonadecensäuremethylester mit 2-Trifluoracetamido-3-carbomethoxymethylamino-3-oxopropylmercaptan und Umwandlung der erhaltenen Verbindung mit einer wäßrigen Base in das gewünschte Produkt.

4. Verfahren nach Anspruch 2 zur Herstellung von 4-Hydroxy-5-[(2-carboxyäthyl)-thio]-6(Z)-nonadecensäure durch Umsetzung von 4,5-Epoxy-6(Z)-nonadecensäuremethylester mit 3-Mercaptopropionsäuremethylester und Umwandlung der erhaltenen Verbindung mit einer wäßrigen Base in das gewünschte Produkt.

5. Verfahren nach Anspruch 1 zur Herstellung der Verbindungen der Formel III

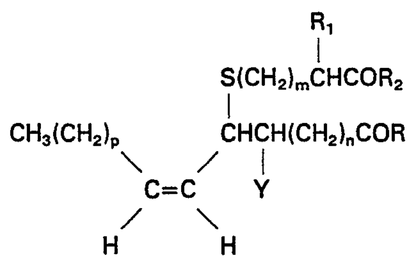

(III)

in der n den Wert 1 hat und Y eine Hydroxylgruppe bedeutet, durch Umsetzung einer Verbindung der Formel

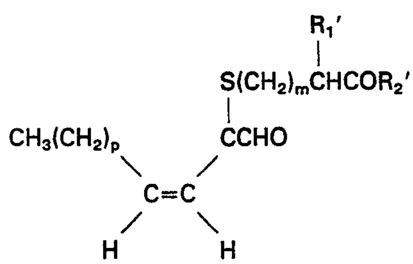

mit $CH_3COR_3$.

6. Verfahren nach Anspruch 5 zur Herstellung von 3-Hydroxy-4-[(2-carboxyäthyl)-thio]-5(Z)-octadecensäure durch Umsetzung von 2-[(2-Carboxyäthyl)-thio]-hexadec-3(Z)-enal mit Essigsäuremethylester und Umwandlung der erhaltenen Verbindung mit einer wäßrigen Base in das gewünschte Produkt.

7. Verfahren nach Anspruch 1 zur Herstellung der Verbindungen der Formel (III)

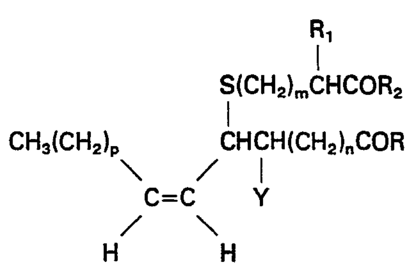

(III)

in der Y ein Wasserstoffatom bedeutet, durch Umsetzung einer Verbindung der Formel

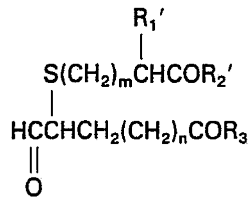

mit einer Verbindung der Formel $CH_3(CH_2)_pCH{=}P\phi_3$, in der $\phi$ eine Phenylgruppe darstellt.

8. Verfahren nach Anspruch 7, zur Herstellung von 4-[(2-Carboxyäthyl)-thio]-5(Z)-octadecensäure durch Umsetzung von 4-[(2-Carbomethoxyäthyl)-thio]-4-formylbuttersäuremethylester mit Tridecyltriphenylphosphonium-ylid und Umwandlung der erhaltenen Verbindung in wäßriger Bas zum gewünschten Produkt.

9. Verfahren zur Herstellung eines Arzneimittels durch Verbinden einer Verbindung der Formel (I) nach einem der Ansprüche 1 bis 8 mit einem pharmazeutisch verträglichen Träger dafür.

**Revendications pour les Etats Contractants: BE, CH, DE, FR, GB, IT, LI, LU, NL, SE**

1. Un composé représenté par la formule (I)

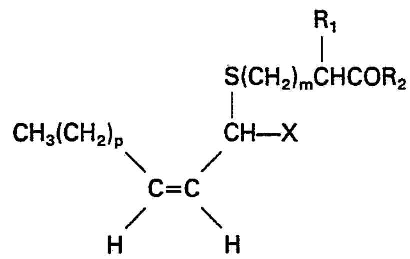

(I)

dans laquelle m est 0, 1 ou 2; p est 9, 10, 11, 12 ou 13; $R_1$ est hydrogène, amino ou

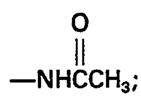;

$R_2$ est hydroxyle, amino, $—NHCH_2CO_2H$,

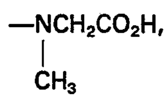, 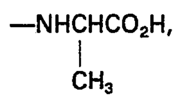, 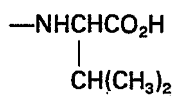

ou $—NHCH_2CONH_2$; et X est $—CO_2H$, $—CH_2OH$,

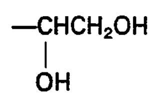

ou bien

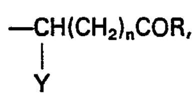

dans laquelle n est 1 ou 2, Y est hydrogène ou hydroxyle et R est hydroxyle ou amino avec la condition que lorsque m est 0, $R_1$ est hydrogène, ou un sel du même pharmaceutiquement acceptable.

2. Un composé de la revendication 1 dans laquelle X est $—CO_2H$, $—CH_2OH$ ou

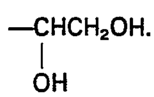

3. Un composé de la revendication 2 qui est l'acide 2-[(2-carboxyéthyle)thio]-3(Z)-hexadécénoique 2-[(2-carboxyéthyle)thio]-3(Z)hexadécen-1-ol ou bien 3-[(2-carboxyéthyle)thio]-4(Z)-heptadécen-1,2-diol.

4. Un composé de la revendication 1 dans laquelle X est

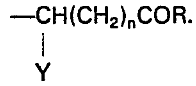

5. Un composé de la revendication 4 dans laquelle R est de l'hydroxyl et Y est de l'hydroxyl.

6. Un composé de la revendication 5 qui est
acide 4-hydroxy-5-[(2-carboxyéthyle)thio]-6(Z)-nonadecenoique;
acide 5-[(3-carboxyméthylamino-3-oxopropyl)-thio]-4-hydroxy-6(Z)-nonadecenoique;
acide 5-[(2-amino-3-carboxyméthylamino-3-oxo-propyl)thio]-4-hydroxy-6(Z)-nonadecenoique;
acide 5-[(3-carboxyméthyle-N-méthylamino-3-oxopropyl)thio]-4-hydroxy-6(Z)-nonadecenoique;
acide 5-[(2-amino-3-carboxamidométhylamino-3-oxo-propyle)thio]-4-hydroxy-6(Z)-nonadecenoique;
acide 4-hydroxy-5-[(carboxyméthyle)thio]-6(Z)-nonadecenoique;
acide 5-[[2-(aminocarbonyle)éthyle]thio]-4-hydroxy-6(Z)-nonadecenoïque;
acide 4-hydroxy-5-[(2-amino-2-carboxyéthyle)thio]-6(Z)-nonadecenoique;
acide 4(R)-hydroxy-5(S)-[(2-amino-2-carboxyéthyle)thio]-6(Z)-nonadecenoique;
acide 4(S)-hydroxy-5(R)-[(2-amino-2-carboxyéthyle)thio]-6(Z)-nonadecenoique; et
acide 4-hydroxy-5-[(3-carboxypropyle)thio]-6(Z)-nonadecenoique.

7. Un composé de la revendication 5 qui est
l'acide 5-[(2-amino-3-carboxyméthylamino-3-oxopropyle)thio]-4-hydroxy-6(Z)-nonadecenoique;
l'acide 5(S)-[(2-amino-3-carboxyméthylamino-3-oxopropyle)thio]-4(R)-hydroxy-6(Z)-nonadecenoique; ou bien
l'acide 5(R)-[(2-amino-3-carboxyméthylamino-3-oxopropyle)thio]-4(S)-hydroxy-6(Z)-nonadecenoique;

8. Un composé de la revendication 5 qui est
acide 3-hydroxy-4-[(2-carboxyéthyle)thio]-5(Z)-octadécenoique,
acide 4-hydroxy-5-[(2-carboxyéthyle)thio]-6(Z)-heptadécenoique, ou bien
acide 4-hydroxy-5[(2-carboxyéthyle)thio-6(Z)-hénéicosénoique.

9. Un composé de la revendication 4 qui est acide 4[(2-carboxoxéthyle)thio]-5(Z)-octadécenoique
acide 5[(2-carboxyéthyle)thio]-6(Z)-nondécenoique,
5[(3-carboxyméthylamino-3-oxopropyl)thio]-4-hydroxy-6(Z)-nonadécénamide, ou bien
4-hydroxy-5-[(2-carboxyéthyle)thio]-6(Z)-nonadécénamide.

10. Une composition pharmaceutique pour l'inhibition des effets des leucotriens sur les sujets ayant besoin d'une telle inhibition, comprenant un support pharmaceutique ou un diluant et une quantité suffisante non toxique afin de produire la dite inhibition d'un composé de la revendication 1.

11. Un composé de la formule (C)

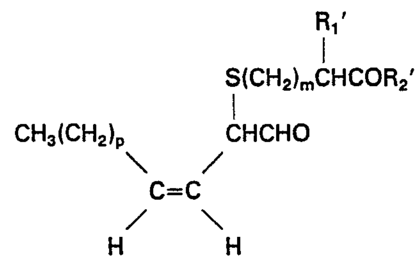

(C)

dans laquelle m est 0, 1 ou 2; p est 9, 10, 11, 12 ou 13; $R_1'$ est hydrogène,

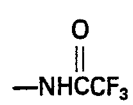 ou 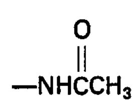

et $R_2'$ est amino, $-NHCH_2CO_2R_3'$,

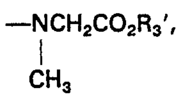, 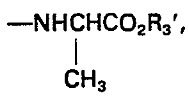, 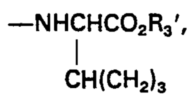,

$-NHCH_2CONH_2$; ou bien $OR_3$, dans lequel $R_3$ est un radical d'alcoyle contenant de un à six atomes de carbones à condition que lorsque m est 0, $R_1$ soit hydrogène.

12. Un procédé pour la préparation des composés représentés par la formule (I)

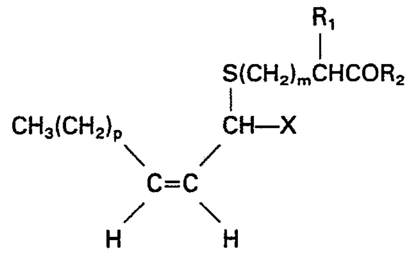

(I)

dans laquelle m est 0, 1 ou 2; p est 9, 10, 11, 12 ou 13; $R_1$ est hydrogène, amino ou

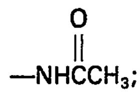

$R_2$ est hydroxyle, amino, $—NHCH_2CO_2H$,

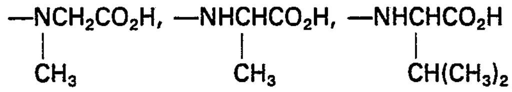

au $—NHCH_2CO\ NH_2$; et X est $—CO_2H$, $—CH_2OH$,

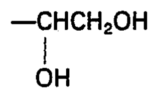

ou bien

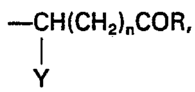

dans laquelle n est 1 ou 2, Y est hydrogène ou hydroxyle et R est hydroxyle ou amino avec la condition que lorsque m est 0, $R_1$ est hydrogène, ou un sel du même pharmaceutiquement acceptable qui comprend:

(A) lorsque X est

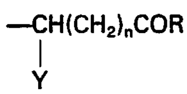

dans laquelle Y est hydroxyl et n est 2, la mise en réaction

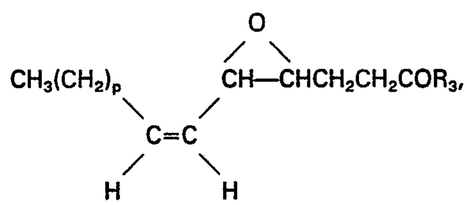

dans lequel $R_3$ est R ou un radical facilement convertissable en R avec

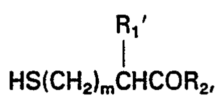

dans laquelle $R_1'$ et $R_2'$ sont respectivement $R_1$ et $R_2$ ou un radical facilement convertissable en $R_1$ et $R_2$;

(B) dans laquelle X est

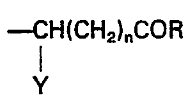

dans laquelle Y est hydroxyle et n est 1, la mise en réaction de

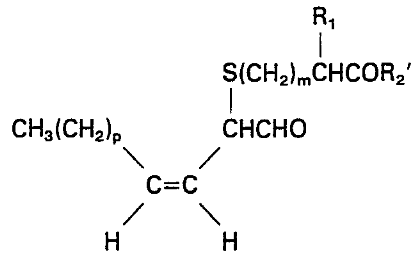

avec $CH_3COR_3$;

(C) lorsque X est

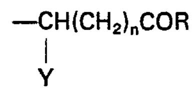

dans laquelle Y est hydrogène,

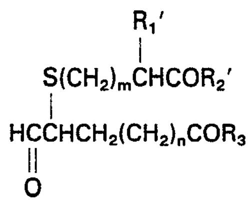

avec $CH_3(CH_2)_pCH{=}P\phi_3$; la réaction de où φ est phényl

(D) lorsque Est $—CO_2H$, la réaction

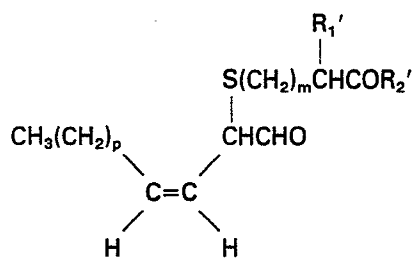

avec un agent oxydant

(E) lorsque X est $—CH_2OH$, la réaction de

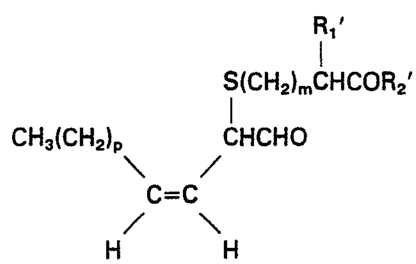

avec un agent de réduction; ou bien

(F) lorsque X est

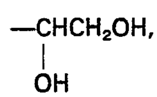

la réaction

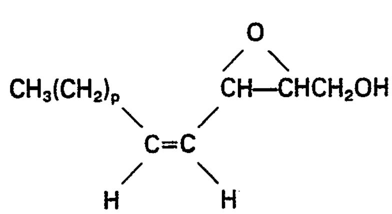

avec

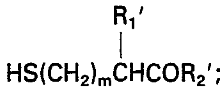

et ensuite en convertissant facultativement $R_1'$, $R_2'$ et $R_3$ en $R_1$, $R_2$ et R et en formant facultativement le sel pharmaceutiquement acceptable des composés.

13. Un composé de la formule (I) comme revendiqué dans n'importe laquelle des revendications de 1 à 9 pour l'emploi en tant que substance thérapeutique active.

14. Un composé de la formule (I) comme revendiqué dans n'importe laquelle des revendications de 1 à 9 pour l'emploi en tant qu'agent anti-asthmatique.

**Revendications pour l'Etat Contractant: AT**

1. Un procédé pour la préparation des composés représentés par la formule (I)

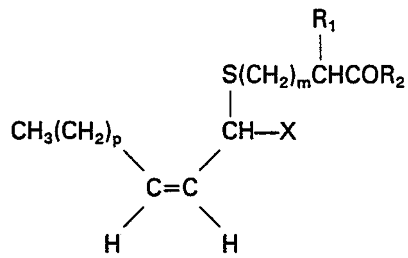

(I)

dans laquelle m est 0, 1 ou 2; p est 9, 10, 11, 12 ou 13; $R_1$ est hydrogène, amino ou 13; $R_1$ est hydrogène, amino ou

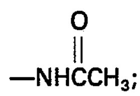

$R_2$ est hydroxyle, amino, $—NHCH_2CO_2H$,

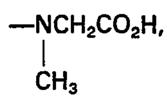 , 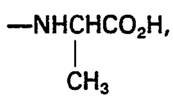 , 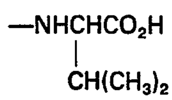

or $—NHCH_2CONH_2$; and X is $—CO_2H$, $—CH_2OH$,

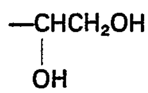

or ou bien

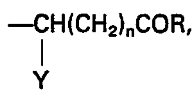

dans laquelle n est 1 ou 2, Y est hydrogène ou hydroxyle et R est hydroxyle ou amino avec la condition que lorsque m est 0, $R_1$ est hydrogène, ou un sel du même pharmaceutiquement acceptable qui comprend:

(A) lorsque X est

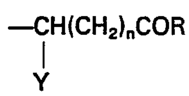

dans laquelle Y est hydroxyl et n est 2, la mise en réaction

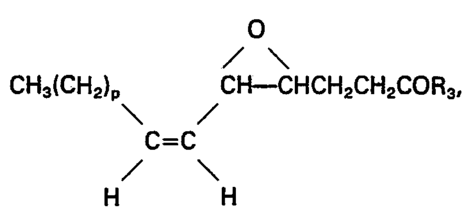

dans lequel $R_3$ est R ou un radical facilement convertissable en R avec

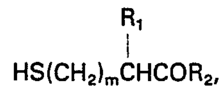

dans laquelle $R_1$, et $R_2$ sont respectivement $R_1$ et $R_2$ ou un radical facilement convertissable en $R_1$ et $R_2$;

(B) dans laquelle X est

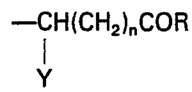

dans laquelle Y est hydroxyl et n est 1, la mise en réaction de

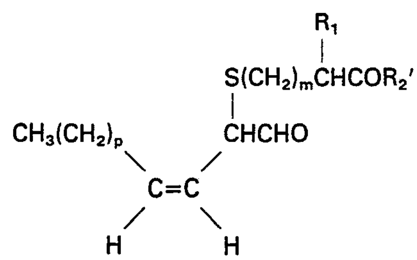

avec $CH_3COR_3$;

(C) lorsque X est

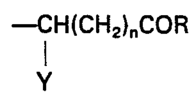

dans laquelle Y est hydrogène, la réaction de

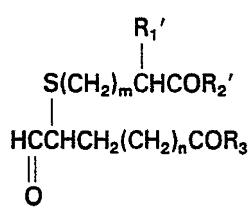

avec $CH_3(CH_2)_pCH{=}P\phi_3$; dans laquelle φ est phenyle

(D) lorsque X est $—CO_2H$, la réaction

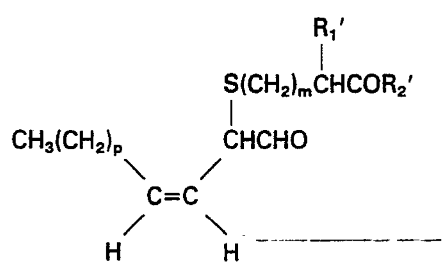

avec un agent oxydant

(E) lorsque X est $—CH_2OH$, la réaction de

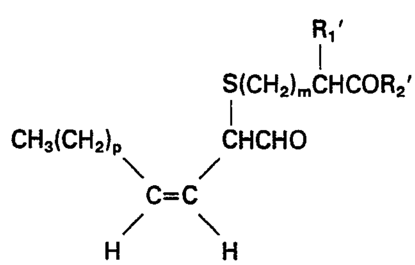

avec un agent de réduction; ou bien

(F) lorsque X est

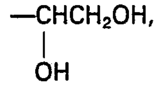

la réaction

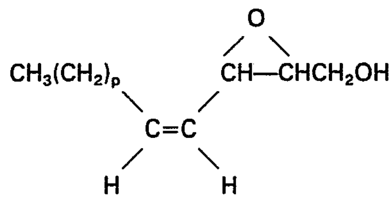

avec

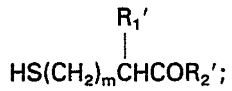

et ensuite en convertissant facultativement $R_1'$, $R_2'$ et $R_3$ en $R_1$, $R_2$ et R et en formant facultativement le sel pharmaceutiquement acceptable des composés.

2. Un procédé selon la revendication 1 pour la préparation des composés de la formule (III)

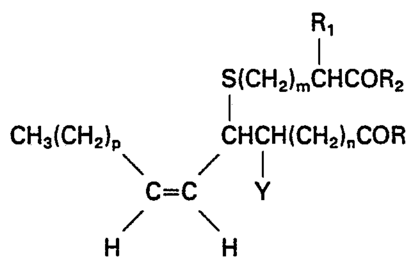

(III)

dans laquelle n est 2 et Y est hydroxyl, qui comprend la réaction de

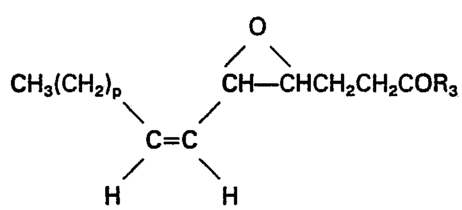

avec

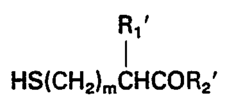

3. Un procédé selon la revendication 2 pour la préparation de l'acide 5-[(2-amino-3-carboxyméthylamino-3-oxopropyl)thio]-4-hydroxy-6(Z)-nonadécénoique, qui comprend la réaction de méthyle 4,5 - époxy - 6(Z) - nonadécénoate avec 2 - trifluoroacétamido - 3 - carbométhoxy - méthylamino-3-oxopropylmercaptan et la conversion du composé en résultant avec la base aqueuse en le produit voulu.

4. Un procédé selon la revendication 2 pour la préparation de l'acide 4-hydroxy-5-[(2-carboxyéthyle)thio]-6(Z)-nonadécénoique, qui comprend la réaction de méthyle 4,5-époxy-6(Z)-nonadécénoate avec du méthyle-3-mercaptopropionate et la conversion du composé en résultant avec la base aqueuse en le produit voulu.

5. Un procédé selon la revendication 1 pour la préparation des composés de la formule (III)

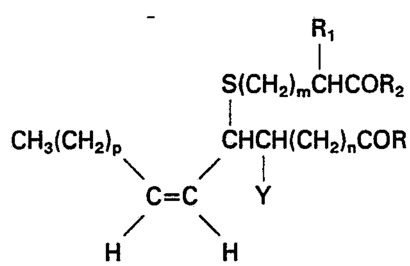

(III)

dans laquelle n est 1 et Y est hydroxyl, qui comprend la réaction de

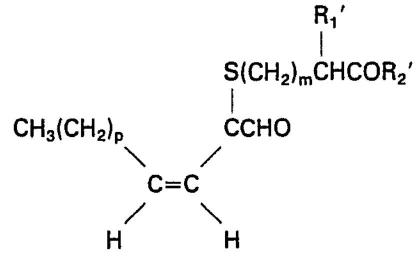

avec $CH_3COR_3$.

6. Un procédé selon la revendication 5 pour la préparation de l'acide 3-hydroxy-4-[(2-carboxyéthyle)thio]-5(Z)-octadécénoique qui comprend la réaction de 2-[(2-carboxyéthyle)thio]-hexadec-3(Z)-énal avec l'acétate de méthyle et la conversion du composé en résultant avec la base aqueuse en le produit voulu.

7. Un procédé selon la revendication 1 pour la préparation des composés de la formule (III)

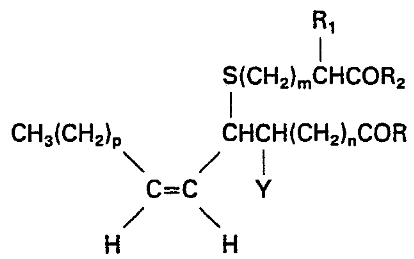

(III)

dans laquelle Y est hydrogène qui comprend la réaction de

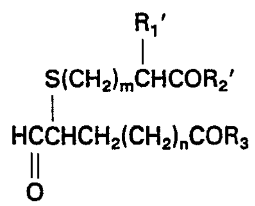

avec $CH_3(CH_2)_pCH{=}P\phi_3$, dans laquelle φ est phényle.

8. Un procédé selon la revendication 7 pour la préparation de l'acide 4-[(2-carboxyéthyle)thio]-5(Z)-octadécénoique qui comprend la réaction du méthyle-4-[(2-carbométhoxyéthyle)thio]-4-formylbutanoate avec du tridécyltriphénylphosphonium ylid et la conversion du composé en résultant avec la base aqueuse en le produit voulu.

9. Un procédé pour la préparation d'une composition pharmaceutique qui comprend la combinaison d'un composé de la formule (I) comme défini dans n'importe laquelle des revendications de 1 à 8 avec un support pharmaceutiquement acceptable du même.